# EUROPEAN PATENT APPLICATION

(11) **EP 4 446 303 A1**
(43) Date of publication of application: **16.10.2024**
(21) Application number: 22907926.4
(22) Date of filing: 14.12.2022
(51) Int. Cl.: C07C 15/28, C09K 11/06, H10K 99/00, H10K 50/00

(54) **ANTHRACENE COMPOUND AND ORGANIC LIGHT-EMITTING DEVICE COMPRISING SAME**

(30) Priority: 14.12.2021 KR 20210178846
(71) Applicant: SFC Co., Ltd., Cheongju-si, Chungcheongbuk-do 28122 (KR)
(72) Inventor: LEE, Se-jin, Cheongju-si Chungcheongbuk-do 28122 (KR); KIM, Si-In, Cheongju-si Chungcheongbuk-do 28122 (KR); PARK, Seok-bae, Cheongju-si Chungcheongbuk-do 28122 (KR); KIM, Hee-dae, Cheongju-si Chungcheongbuk-do 28122 (KR); CHOI, Yeong-tae, Cheongju-si Chungcheongbuk-do 28122 (KR); KIM, Kyung-tae, Cheongju-si Chungcheongbuk-do 28122 (KR); KIM, Ji-yung, Cheongju-si Chungcheongbuk-do 28122 (KR); LEE, Seung-soo, Cheongju-si Chungcheongbuk-do 28122 (KR); KIM, Kyeong-hyeon, Cheongju-si Chungcheongbuk-do 28122 (KR); LEE, Tae-gyun, Cheongju-si Chungcheongbuk-do 28122 (KR); KIM, Joon-ho, Cheongju-si Chungcheongbuk-do 28122 (KR)
(74) Representative: Grosse, Felix Christopher
(86) International application number: PCT/KR2022/020334
(87) International publication number: WO 2023/113459

(57) **Abstract**

The present invention relates to an anthracene derivative compound having a characteristic structure in which an aryl group is introduced into a skeletal structure in which deuterium-substituted phenyl and anthracene are linked. In addition, the present invention relates to a high-efficiency, long-life organic light-emitting device of which the luminous efficiency and lifetime characteristics are significantly improved by employing a polycyclic compound having a characteristic structure as a dopant for a light-emitting layer while employing said compound as a host for the light-emitting layer. Furthermore, the present invention relates to a high-efficiency, long-life organic light-emitting device of which the luminous efficiency and lifetime characteristics are significantly improved by employing a polycyclic compound having a characteristic structure as a dopant for a light-emitting layer while forming a host of the light-emitting layer with a plurality of materials of one or more characteristic anthracene compounds in addition to said compound.

## Description

### Technical Field

The present invention relates to an anthracene derivative having a specific structure in which an aryl moiety is introduced to a skeletal structure containing a deuterated phenyl moiety and an anthracene moiety linked to the deuterated phenyl moiety. The present invention also relates to a highly efficient and long-lasting organic light emitting device which includes a light emitting layer employing the anthracene derivative as a host and a polycyclic compound with a specific structure as a dopant, achieving significantly improved luminous efficiency and life characteristics.

### Background Art

Organic light emitting devices are self-luminous devices in which electrons injected from an electron injecting electrode (cathode) recombine with holes injected from a hole injecting electrode (anode) in a light emitting layer to form excitons, which emit light while releasing energy. Such organic light emitting devices have the advantages of low driving voltage, high luminance, large viewing angle, and short response time and can be applied to full-color light emitting flat panel displays. Due to these advantages, organic light emitting devices have received attention as nextgeneration light sources.

The above characteristics of organic light emitting devices are achieved by structural optimization of organic layers of the devices and are supported by stable and efficient materials for the organic layers, such as hole injecting materials, hole transport materials, light emitting materials, electron transport materials, electron injecting materials, and electron blocking materials. However, more research still needs to be done to develop structurally optimized structures of organic layers for organic light emitting devices and stable and efficient materials for organic layers of organic light emitting devices.

Particularly, for maximum efficiency in a light emitting layer, an appropriate combination of energy band gaps of a host and a dopant is required such that holes and electrons migrate to the dopant through stable electrochemical paths to form excitons.

### Detailed Description of the Invention

### Problems to be Solved by the Invention

Therefore, the present invention is intended to provide a host material for a light emitting layer of an organic light emitting device that has a specific structure in which an aryl moiety is introduced to a skeletal structure containing a deuterated phenyl moiety and an anthracene moiety linked to the deuterated phenyl moiety. The present invention is also intended to provide a highly efficient and long-lasting organic light emitting device that employs the host material to achieve significantly improved luminous efficiency and life characteristics.

### Means for Solving the Problems

One aspect of the present invention provides an anthracene derivative as a host compound for an organic layer (preferably a light emitting layer) of a device, represented by Formula A:

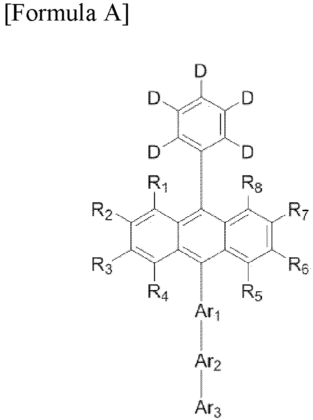

The specific structure of Formula A, definitions of the substituents in Formula A, and specific compounds that can be represented by Formula A are described below.

A further aspect of the present invention provides an organic light emitting device including a first electrode, a second electrode opposite to the first electrode, and a light emitting layer interposed between the first and second electrodes wherein the light emitting layer includes a host mixture of an anthracene derivative represented by Formula A: 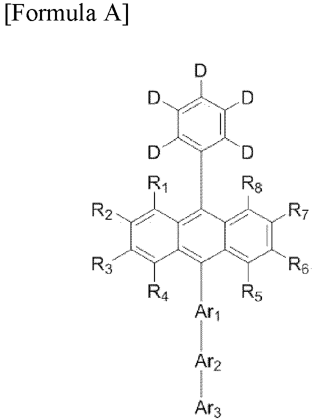 an anthracene derivative represented by Formula B:

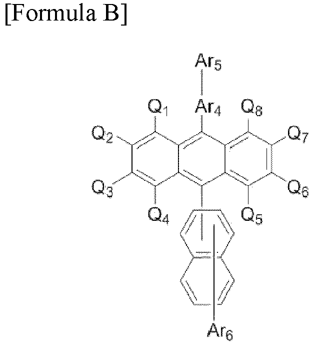

The specific structures of Formulas A and B, definitions of the substituents in Formulas A and B, and specific compounds that can be represented by Formulas A and B are described below.

According to one embodiment of the present invention, the light emitting layer of the organic light emitting device may employ the anthracene compound represented by Formula A and/or Formula B as a host and a compound represented by Formula 1 as a dopant:

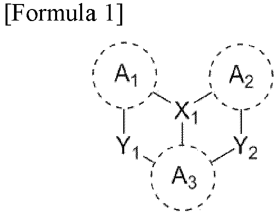

The use of the dopant ensures significantly improved luminous efficiency and life characteristics of the organic light emitting device and makes the device highly efficient and long lasting.

The specific structure of Formula 1, definitions of the substituents in Formula 1, and specific compounds that can be represented by Formula 1 are described below.

### Effects of the Invention

The organic light emitting device of the present invention includes a light emitting layer employing, as a host, the anthracene derivative having a specific structure in which an aryl moiety is introduced to a skeletal structure containing a deuterated phenyl moiety and an anthracene moiety linked to the deuterated phenyl moiety. The use of the host ensures high luminous efficiency and improved life characteristics of the device. Due to these advantages, the highly efficient and long-lasting organic light emitting device can find applications in not only lighting systems but also a variety of displays, including flat panel displays, flexible displays, and wearable displays.

### Best Mode for Carrying out the Invention

The present invention will now be described in more detail.

One aspect of the present invention is directed to an anthracene derivative represented by Formula A: 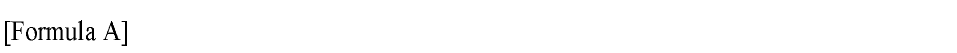 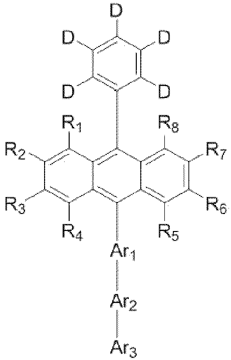 wherein R₁ to R₈ are identical to or different from each other and are each independently hydrogen or deuterium and Ar₁ to Ar₃ are identical to or different from each other and are each independently substituted or unsubstituted C₆-C₅₀ aryl or substituted or unsubstituted C₃-C₅₀ mixed aliphatic-aromatic cyclic group.

According to one embodiment of the present invention, at least four of R₁ to R₈ may be deuterium.

According to one embodiment of the present invention, each of Ar₁ to Ar₃ may be independently substituted or unsubstituted C₆-C₂₄ aryl that are deuterated.

According to one embodiment of the present invention, each of Ar₁ to Ar₃ may be selected from Structural Formulas 1 to 3: 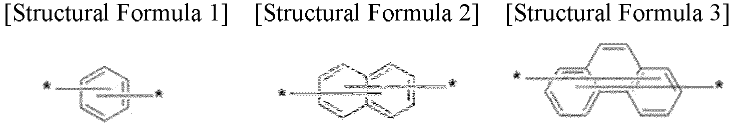 wherein each of the non-bonded carbon atoms is optionally replaced by a deuterium atom.

The term "substituted" in the definitions of Ar₁ to Ar₃ in Formula A indicates substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof. The term "unsubstituted" in the same definition indicates having no substituent. One or more hydrogen atoms in each of the substituents are optionally replaced by deuterium atoms.

The anthracene derivative of the present invention has a structure in which an aryl moiety is introduced to a skeletal structure containing a deuterated phenyl moiety and an anthracene moiety linked to the deuterated phenyl moiety. Due to this structure, the anthracene derivative of the present invention can be employed as a host compound in a light emitting layer of an organic light emitting device. The use of the anthracene derivative makes the organic light emitting device highly efficient and long lasting.

A further aspect of the present invention is directed to an organic light emitting device including a first electrode, a second electrode, and one or more organic layers interposed between the first and second electrodes wherein one of the organic layers, preferably a light emitting layer includes the compound represented by Formula A.

According to one embodiment of the present invention, the organic layer may include, as hosts, at least one anthracene compound represented by Formula A: 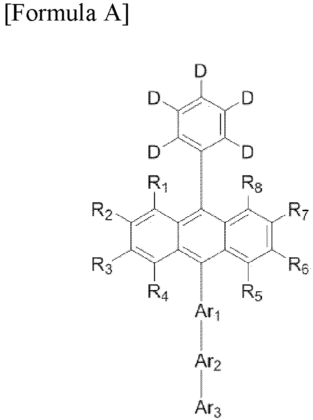
wherein R₁ to R₈ are identical to or different from each other and are each independently hydrogen or deuterium and Ar₁ to Ar₃ are identical to or different from each other and are each independently substituted or unsubstituted C₆-C₅₀ aryl or substituted or unsubstituted C₃-C₅₀ mixed aliphatic-aromatic cyclic group; and
at least one anthracene compound represented by Formula B: 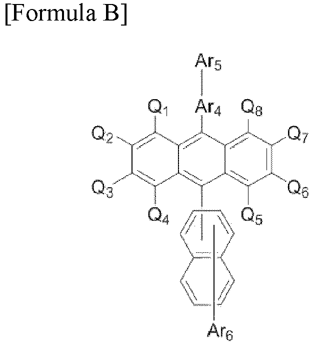 wherein Ar₄ to Ar₆ are identical to or different from each other and are each independently selected from hydrogen, deuterium, substituted or unsubstituted C₁-C₃₀ alkyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted amine, substituted or unsubstituted silyl, substituted or unsubstituted germanium, nitro, cyano, and halogen, and Q₁ to Q₈ are identical to or different from each other and are each independently hydrogen or deuterium, with the proviso that at least one of Q₁ to Q₈ is deuterium.

The term "substituted" in the definitions of Ar₁ to Ar₃ in Formula A and Ar₄ to Ar₆ in Formula B indicates substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof. The term "unsubstituted" in the same definition indicates having no substituent. One or more hydrogen atoms in each of the substituents are optionally replaced by deuterium atoms.

According to one embodiment of the present invention, the light emitting layer of the organic light emitting device may include the anthracene derivative represented by Formula A and/or B as a host and a polycyclic compound represented by Formula 1 as a dopant: 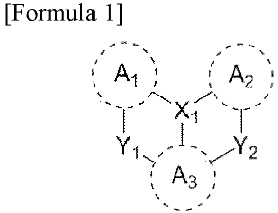 wherein X₁ is B, P=O, P=S or Al, Y₁ and Y₂ are identical to or different from each other and are each independently selected from NR₁₁, O, S, CR₁₂R₁₃, SiR₁₄R₁₅ or GeR₁₆R₁₇, A₁ to A₃ are identical to or different from each other and are each independently selected from substituted or unsubstituted C₆-C₅₀ aromatic hydrocarbon rings, substituted or unsubstituted C₃-C₅₀ aliphatic hydrocarbon rings, substituted or unsubstituted C₂-C₅₀ aromatic heterocyclic rings, substituted or unsubstituted C₂-C₅₀ aliphatic heterocyclic rings, and substituted or unsubstituted C₃-C₅₀ mixed aliphatic-aromatic cyclic group, R₁₁ to R₁₇ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, with the proviso that each of R₁₁ to R₁₇ is optionally linked to one or more of the rings A₁ to A₃ to form an alicyclic or aromatic monocyclic or polycyclic ring, R₁₂ and R₁₃ are optionally linked to each other to form an alicyclic or aromatic monocyclic or polycyclic ring, R₁₄ and R₁₅ are optionally linked to each other to form an alicyclic or aromatic monocyclic or polycyclic ring, and R₁₆ and R₁₇ are optionally linked to each other to form an alicyclic or aromatic monocyclic or polycyclic ring.

The term "substituted" in the definitions of Y₁, Y₂, and A₁ to A₃ in Formula 1 indicates substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof. The term "unsubstituted" in the same definition indicates having no substituent. One or more hydrogen atoms in each of the substituents are optionally replaced by deuterium atoms.

According to one embodiment of the present invention, the compound represented by Formula 1 may be a polycyclic compound represented by Formula 2 or 3: 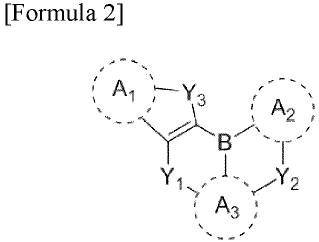 wherein Y₃ is O, S or NR₁₈, R₁₈ is as defined for R₁₁ to R₁₇ in Formula 1, and Y₁, Y₂, and A₁ to A₃ are as defined in Formula 1; 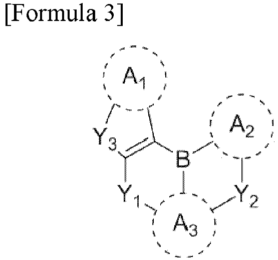 wherein Y₃ is O, S or NR₁₈, R₁₈ is as defined for R₁₁ to R₁₇ in Formula 1, and Y₁, Y₂, and A₁ to A₃ are as defined in Formula 1.

According to one embodiment of the present invention, the compound represented by Formula 1 may be selected from polycyclic compounds represented by Formulas 2-1, 2-2, 2-3, 3-1, 3-2, and 3-3: 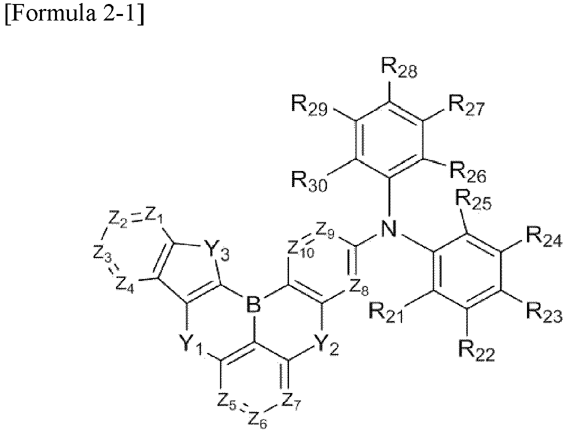 wherein Z₁ to Z₁₀ are identical to or different from each other and are each independently CR₃₁ or N, provided that when two or more of Z₁ to Z₁₀ are CR₃₁, the moieties CR₃₁ are identical to or different from each other and adjacent ones of the groups R₃₁ are optionally linked to each other to form an alicyclic or aromatic monocyclic or polycyclic ring, Y₃ is O, S or NR₁₈, R₁₈ is as defined for R₁₁ to R₁₇ in Formula 1, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, R₂₁ to R₃₀ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₂₁ to R₃₁ are optionally replaced by deuterium atoms, adjacent ones of the substituents of R₂₁ to R₃₀ are optionally linked to each other to form an alicyclic or aromatic monocyclic or polycyclic ring, and the groups R₃₁ are optionally linked to each other to form an alicyclic or aromatic monocyclic or polycyclic ring, provided that when Z₈ and Z₉ are CR₃₁, each of R₂₁, R₂₅, R₂₆, and R₃₀ adjacent to Z₈ and Z₉ is optionally bonded to R₃₁ to form an alicyclic or aromatic monocyclic or polycyclic ring, Y₁ and Y₂ are identical to or different from each other and are each independently as defined in Formula 1 or are each independently a linker represented by Structural Formula A: 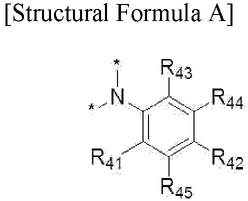 wherein R₄₁ to R₄₅ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₄₁ to R₄₅ are optionally replaced by deuterium atoms and each of R₄₁ to R₄₅ is optionally linked to one or more adjacent substituents to form an alicyclic or aromatic monocyclic or polycyclic ring; 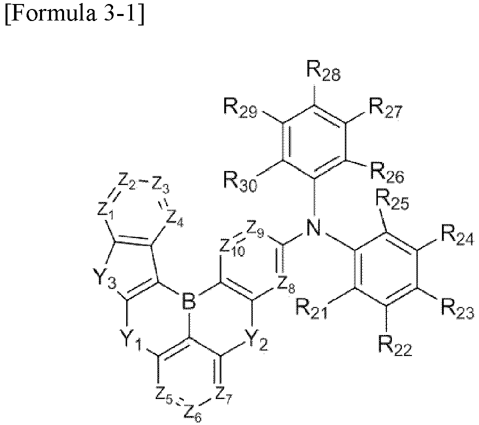 wherein Z₁ to Z₁₀, Y₁ to Y₃, and R₂₁ to R₃₀ are as defined in Formula 2-1; 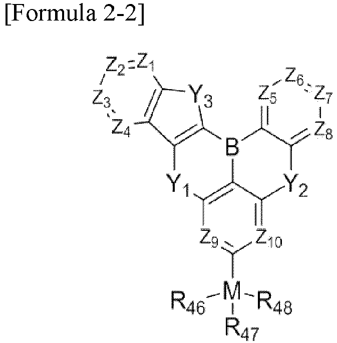 wherein Z₁ to Z₁₀ are identical to or different from each other and are each independently CR₃₁ or N, provided that when two or more of Z₁ to Z₁₀ are CR₃₁, adjacent ones of the moieties CR₃₁ are identical to or different from each other, Y₃ is O, S or NR₁₈, R₁₈ is as defined for R₁₁ to R₁₇ in Formula 1, M is Si or Ge, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, R₄₆ to R₄₈ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₃₁ and R₄₆ to R₄₈ are optionally replaced by deuterium atoms and the substituents of R₄₆ to R₄₈ are optionally linked to each other to form an alicyclic or aromatic monocyclic or polycyclic ring; 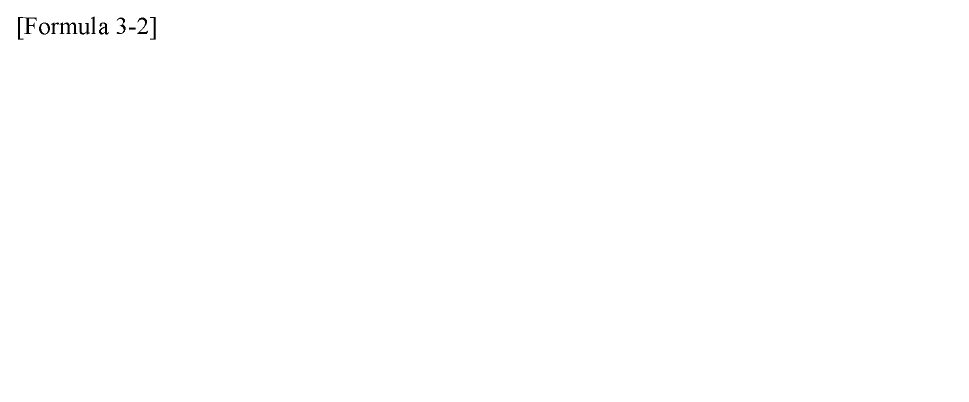 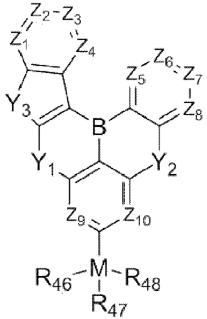 wherein Z₁ to Z₁₀, Y₁ to Y₃, M, and R₄₆ to R₄₈ are as defined in Formula 2-2; 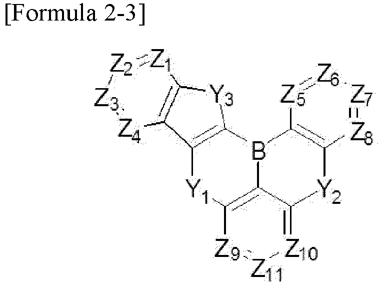 wherein Z₁ to Z₁₁ are identical to or different from each other and are each independently CR₃₁ or N, provided that when two or more of Z₁ to Z₁₁ are CR₃₁, the moieties CR₃₁ are identical to or different from each other and adjacent ones of the groups R₃₁ are optionally linked to each other to form an alicyclic or aromatic monocyclic or polycyclic ring, Y₃ is O, S or NR₁₈, R₁₈ is as defined for R₁₁ to R₁₇ in Formula 1, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, with the proviso that one or more hydrogen atoms in each of the substituents of the groups R₃₁ are optionally replaced by deuterium atoms, Y₁ and Y₂ are identical to or different from each other and are each independently as defined in Formula 1 or are each independently a linker represented by Structural Formula B: 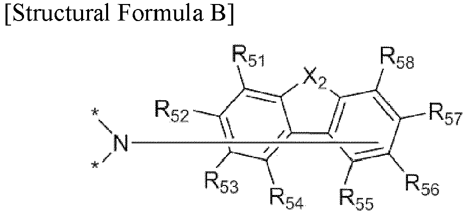 wherein X₂ is O or S, R₅₁ to R₅₈ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₅₁ to R₅₈ are optionally replaced by deuterium atoms, one of R₅₁ to R₅₈ forms a single bond with the nitrogen atom, and each of the others is optionally linked to one or more adjacent substituents to form an alicyclic or aromatic monocyclic or polycyclic ring; and 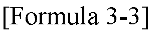 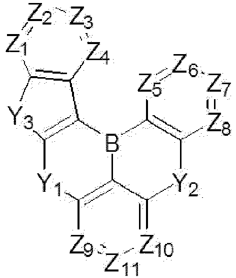 wherein Z₁ to Z₁₁ and Y₁ to Y₃ are as defined in Formula 2-3.

The term "substituted" in the definitions of Z₁ to Z₁₀, Y₁ to Y₃, and R₂₁ to R₃₀ in Formula 2-1, Z₁ to Z₁₀, Y₁ to Y₃, and R₄₆ to R₄₈ in Formula 2-2, Z₁ to Z₁₁ and Y₁ to Y₃ in Formula 2-3, Z₁ to Z₁₀, Y₁ to Y₃, and R₂₁ to R₃₀ in Formula 3-1, Z₁ to Z₁₀, Y₁ to Y₃, and R₄₆ to R₄₈ in Formula 3-2, and Z₁ to Z₁₁ and Y₁ to Y₃ in Formula 3-3 indicates substitution with one or more substituents selected from deuterium, C₁-C₂₈ alkyl, C₁-C₂₈ haloalkyl, C₃-C₁₈ cycloalkyl, C₂-C₁₈ alkenyl, C₂-C₁₈ alkynyl, C₁-C₂₈ heteroalkyl, C₂-C₁₈ heterocycloalkyl, C₆-C₂₄ aryl, C₇-C₂₄ arylalkyl, C₇-C₂₄ alkylaryl, C₂-C₂₄ heteroaryl, C₃-C₂₄ heteroarylalkyl, C₃-C₂₄ alkylheteroaryl, C₃-C₂₄ mixed aliphatic-aromatic cyclic groups, C₁-C₁₈ alkoxy, C₆-C₁₈ aryloxy, C₆-C₁₈ arylthionyl, C₁-C₃₀ amine, C₁-C₃₀ silyl, C₁-C₃₀ germanium, cyano, halogen, hydroxyl, and nitro, or a combination thereof. The term "unsubstituted" in the same definition indicates having no substituent. One or more hydrogen atoms in each of the substituents are optionally replaced by deuterium atoms.

The content of the dopant in the light emitting layer is typically in the range of about 0.01 to about 20 parts by weight, based on about 100 parts by weight of the host but is not limited to this range.

The light emitting layer may further include one or more dopants other than the polycyclic compound represented by Formula 1 and one or more hosts other than the compound represented by Formula A and/or B. In this case, the hosts and the dopants may be mixed or stacked in the light emitting layer.

In the "substituted or unsubstituted C₁-C₃₀ alkyl", "substituted or unsubstituted C₆-C₅₀ aryl", etc., the number of carbon atoms in the alkyl or aryl group indicates the number of carbon atoms constituting the unsubstituted alkyl or aryl moiety without considering the number of carbon atoms in the substituent(s). For example, a phenyl group substituted with a butyl group at the para-position corresponds to a C₆ aryl group substituted with a C₄ butyl group.

As used herein, the expression "optionally linked to each other or an adjacent group to form a ring" means that the corresponding adjacent substituents are bonded to each other or each of the corresponding substituents is bonded to an adjacent group to form a substituted or unsubstituted alicyclic or aromatic ring. The term "adjacent group" may mean a substituent on an atom directly attached to an atom substituted with the corresponding substituent, a substituent disposed sterically closest to the corresponding substituent or another substituent on an atom substituted with the corresponding substituent. For example, two substituents substituted at the ortho position of a benzene ring or two substituents on the same carbon in an aliphatic ring may be considered "adjacent" to each other. Optionally, the paired substituents each lose one hydrogen radical and are linked to each other to form a ring. The carbon atoms in the resulting alicyclic, aromatic mono- or polycyclic ring may be replaced by one or more heteroatoms such as N, NR (wherein R is as defined for R₁₁ to R₁₇), O, S, Si, and Ge.

In the present invention, the alkyl groups may be straight or branched. Specific examples of the alkyl groups include, but are not limited to, methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 2-methylpentyl, 4-methylhexyl, and 5-methylhexyl groups.

In the present invention, specific examples of the arylalkyl groups include, but are not limited to, phenylmethyl(benzyl), phenylethyl, phenylpropyl, naphthylmethyl, and naphthylethyl.

In the present invention, specific examples of the alkylaryl groups include, but are not limited to, tolyl, xylenyl, dimethylnaphthyl, t-butylphenyl, t-butylnaphthyl, and t-butylphenanthryl.

The alkenyl group is intended to include straight and branched ones and may be optionally substituted with one or more other substituents. The alkenyl group may be specifically a vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, stilbenyl or styrenyl group but is not limited thereto.

The alkynyl group is intended to include straight and branched ones and may be optionally substituted with one or more other substituents. The alkynyl group may be, for example, ethynyl or 2-propynyl but is not limited thereto.

The cycloalkenyl group is a non-aromatic cyclic unsaturated hydrocarbon group having one or more carbon-carbon double bonds. The cycloalkenyl group may be, for example, cyclopropenyl, cyclobutenyl, cyclopentenyl, cyclohexenyl, cycloheptenyl, 1,3-cyclohexadienyl, 1,4-cyclohexadienyl, 2,4-cycloheptadienyl or 1,5-cyclooctadienyl but is not limited thereto.

The aromatic hydrocarbon rings or aryl groups may be monocyclic or polycyclic ones. As used herein, the term "polycyclic" means that the aromatic hydrocarbon ring may be directly attached or fused to one or more other cyclic groups. The other cyclic groups may be aromatic hydrocarbon rings and other examples thereof include aliphatic heterocyclic rings, aliphatic hydrocarbon rings, and aromatic heterocyclic rings. Examples of the monocyclic aryl groups include, but are not limited to, phenyl, biphenyl, and terphenyl. Examples of the polycyclic aryl groups include naphthyl, anthracenyl, phenanthrenyl, pyrenyl, perylenyl, tetracenyl, chrysenyl, fluorenyl, acenaphathcenyl, triphenylene, and fluoranthrene groups but the scope of the present invention is not limited thereto.

The aromatic heterocyclic rings or heteroaryl groups refer to aromatic groups containing one or more heteroatoms. Examples of the aromatic heterocyclic rings or heteroaryl groups include, but are not limited to, thiophene, furan, pyrrole, imidazole, thiazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, and phenothiazinyl groups.

The aliphatic hydrocarbon rings or cycloalkyl groups refer to non-aromatic rings consisting only of carbon and hydrogen atoms. The aliphatic hydrocarbon ring is intended to include monocyclic and polycyclic ones and may be optionally substituted with one or more other substituents. As used herein, the term "polycyclic" means that the aliphatic hydrocarbon ring may be directly attached or fused to one or more other cyclic groups. The other cyclic groups may be aliphatic hydrocarbon rings and other examples thereof include aliphatic heterocyclic rings, aromatic hydrocarbon rings, and aromatic heterocyclic rings. Specific examples of the aliphatic hydrocarbon rings include, but are not limited to, cycloalkyl groups such as cyclopropyl, cyclobutyl, cyclopentyl, adamantyl, bicycloheptanyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl, cycloalkanes such as cyclohexane and cyclopentane, and cycloalkenes such as cyclohexene and cyclobutene.

The aliphatic heterocyclic rings or heterocycloalkyl groups refer to aliphatic rings containing one or more heteroatoms such as O, S, Se, N, and Si. The aliphatic heterocyclic ring is intended to include monocyclic or polycyclic ones and may be optionally substituted with one or more other substituents. As used herein, the term "polycyclic" means that the aliphatic heterocyclic ring such as heterocycloalkyl or heterocycloalkane may be directly attached or fused to one or more other cyclic groups. The other cyclic groups may be aliphatic heterocyclic rings and other examples thereof include aliphatic hydrocarbon rings, aromatic hydrocarbon rings, and aromatic heterocyclic rings.

The mixed aliphatic-aromatic cyclic group refers to a ring in which an aliphatic ring and an aromatic ring are linked and fused together and which are overall non-aromatic. More specifically, the mixed aliphatic-aromatic cyclic group may be an aromatic hydrocarbon cyclic group fused with an aliphatic hydrocarbon ring, an aromatic hydrocarbon cyclic group fused with an aliphatic heterocyclic ring, an aromatic heterocyclic group fused with an aliphatic hydrocarbon ring, an aromatic heterocyclic group fused with an aliphatic heterocyclic ring, an aliphatic hydrocarbon cyclic group fused with an aromatic hydrocarbon ring, an aliphatic hydrocarbon cyclic group fused with an aromatic hydrocarbon ring, an aliphatic heterocyclic group fused with an aromatic hydrocarbon ring or an aliphatic heterocyclic group fused with an aromatic heterocyclic ring. Specific examples of such mixed aliphatic-aromatic cyclic groups include tetrahydronaphthyl, tetrahydrobenzocycloheptene, tetrahydrophenanthrene, tetrahydroanthracenyl, octahydrotriphenylene, tetrahydrobenzothiophene, tetrahydrobenzofuranyl, tetrahydrocarbazole, and tetrahydroquinoline. The mixed aliphatic-aromatic cyclic group may be interrupted by at least one heteroatom other than carbon. The heteroatom may be, for example, N, O, S, Si, Ge or P.

The alkoxy group may be specifically a methoxy, ethoxy, propoxy, isobutyloxy, sec-butyloxy, pentyloxy, iso-amyloxy or hexyloxy group but is not limited thereto.

The silyl group is intended to include -SiH₃, alkylsilyl, arylsilyl, alkylarylsilyl, arylheteroarylsilyl, and heteroaryl silyl. The arylsilyl refers to a silyl group obtained by substituting one, two or three of the hydrogen atoms in -SiH₃ with aryl groups. The alkylsilyl refers to a silyl group obtained by substituting one, two or three of the hydrogen atoms in -SiH₃ with alkyl groups. The alkylarylsilyl refers to a silyl group obtained by substituting one of the hydrogen atoms in -SiH₃ with an alkyl group and the other two hydrogen atoms with aryl groups or substituting two of the hydrogen atoms in -SiH₃ with alkyl groups and the remaining hydrogen atom with an aryl group. The arylheteroaryl silyl refers to a silyl group obtained by substituting one of the hydrogen atoms in -SiH₃ with an aryl group and the other two hydrogen atoms with heteroaryl groups or substituting two of the hydrogen atoms in -SiH₃ with aryl groups and the remaining hydrogen atom with a heteroaryl group. The heteroarylsilyl refers to a silyl group obtained by substituting one, two or three of the hydrogen atoms in -SiH₃ with heteroaryl groups. The arylsilyl group may be, for example, substituted or unsubstituted monoarylsilyl, substituted or unsubstituted diarylsilyl, or substituted or unsubstituted triarylsilyl. The same applies to the alkylsilyl and heteroarylsilyl groups.

Each of the aryl groups in the aryl silyl, heteroarylsilyl, and arylheteroarylsilyl groups may be a monocyclic or polycyclic one. Each of the heteroaryl groups in the arylsilyl, heteroarylsilyl, and arylheteroarylsilyl groups may be a monocyclic or polycyclic one.

Specific examples of the silyl groups include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl. One or more of the hydrogen atoms in each of the silyl groups may be substituted with the substituents mentioned in the aryl groups.

The amine group is intended to include -NH₂, alkylamine, arylamine, arylheteroarylamine, and heteroarylamine. The arylamine refers to an amine group obtained by substituting one or two of the hydrogen atoms in -NH₂ with aryl groups. The alkylamine refers to an amine group obtained by substituting one or two of the hydrogen atoms in -NH₂ with alkyl groups. The alkylarylamine refers to an amine group obtained by substituting one of the hydrogen atoms in -NH₂ with an alkyl group and the other hydrogen atom with an aryl group. The arylheteroarylamine refers to an amine group obtained by substituting one of the hydrogen atoms in -NH₂ with an aryl group and the other hydrogen atom with a heteroaryl group. The heteroarylamine refers to an amine group obtained by substituting one or two of the hydrogen atoms in -NH₂ with heteroaryl groups. The arylamine may be, for example, substituted or unsubstituted monoarylamine, substituted or unsubstituted diarylamine, or substituted or unsubstituted triarylamine. The same applies to the alkylamine and heteroarylamine groups.

Each of the aryl groups in the arylamine, heteroarylamine, and arylheteroarylamine groups may be a monocyclic or polycyclic one. Each of the heteroaryl groups in the arylamine, heteroarylamine, and arylheteroarylamine groups may be a monocyclic or polycyclic one.

The germanium group is intended to include -GeH₃, alkylgermanium, arylgermanium, heteroarylgermanium, alkylarylgermanium, alkylheteroarylgermanium, and arylheteroarylgermanium. The definitions of the substituents in the germanium groups follow those described for the silyl groups, except that the silicon (Si) atom in each silyl group is changed to a germanium (Ge) atom.

Specific examples of the germanium groups include trimethylgermane, triethylgermane, triphenylgermane, trimethoxygermane, dimethoxyphenylgermane, diphenylmethylgermane, diphenylvinylgermane, methylcyclobutylgermane, and dimethylfurylgermane. One or more of the hydrogen atoms in each of the germanium groups may be substituted with the substituents mentioned in the aryl groups.

The cycloalkyl, aryl, and heteroaryl groups in the cycloalkyloxy, aryloxy, heteroaryloxy, cycloalkylthioxy, arylthioxy, and heteroarylthioxy groups are the same as those exemplified above. Specific examples of the aryloxy groups include, but are not limited to, phenoxy, p-tolyloxy, m-tolyloxy, 3,5-dimethylphenoxy, 2,4,6-trimethylphenoxy, p-tert-butylphenoxy, 3-biphenyloxy, 4-biphenyloxy, 1-naphthyloxy, 2-naphthyloxy, 4-methyl-1-naphthyloxy, 5-methyl-2-naphthyloxy, 1-anthryloxy, 2-anthryloxy, 9-anthryloxy, 1-phenanthryloxy, 3-phenanthryloxy, and 9-phenanthryloxy groups. Specific examples of the arylthioxy groups include, but are not limited to, phenylthioxy, 2-methylphenylthioxy, and 4-tert-butylphenylthioxy groups.

The halogen group may be, for example, fluorine, chlorine, bromine or iodine.

According to one embodiment of the present invention, the anthracene derivative represented by Formula A may be selected from the following compounds:

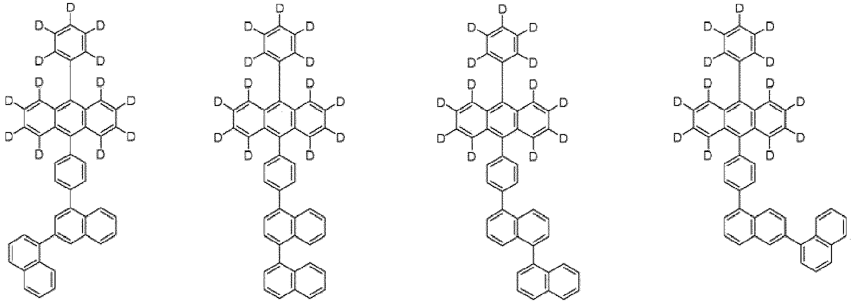

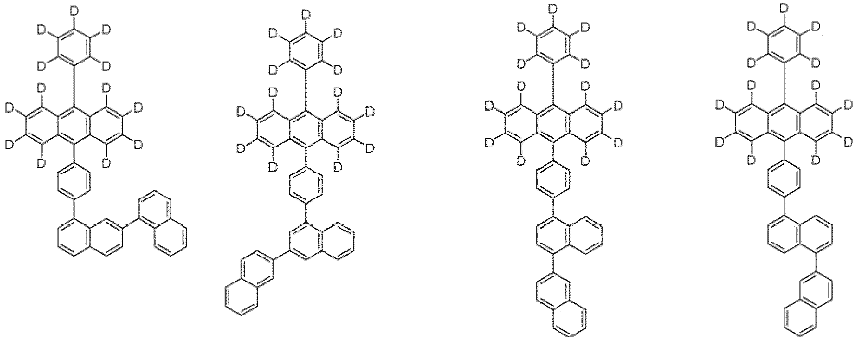

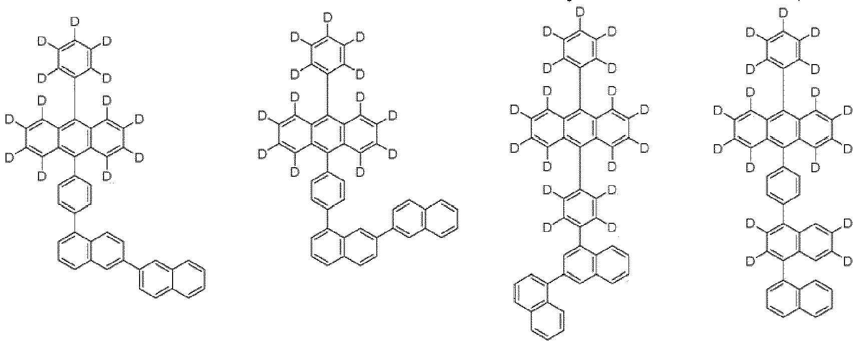

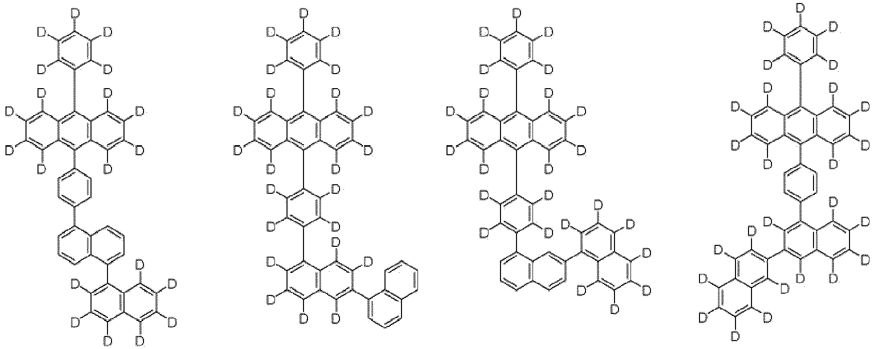

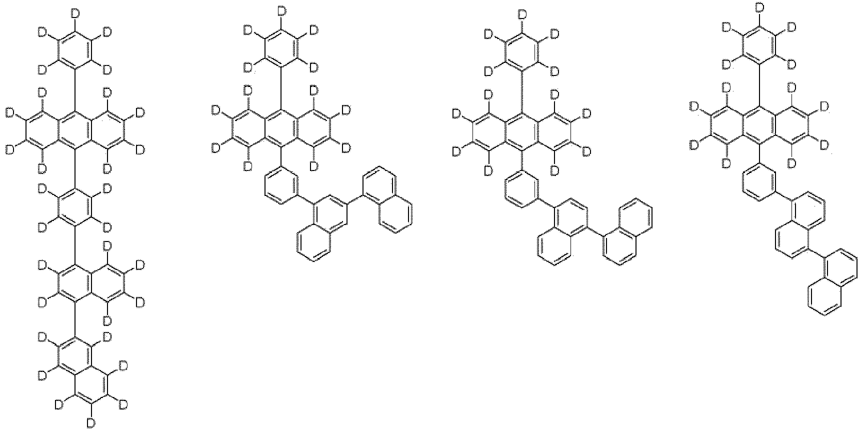

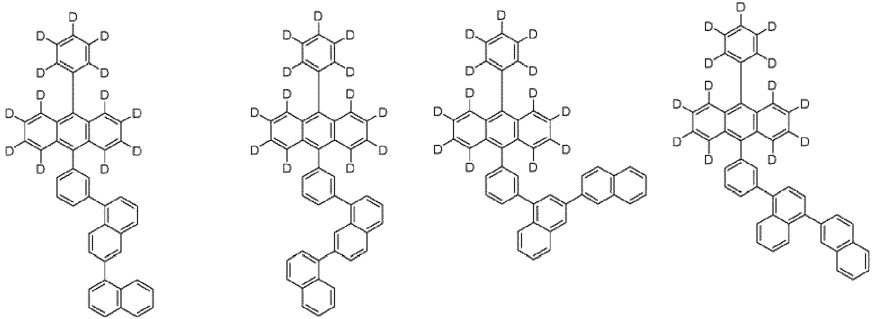

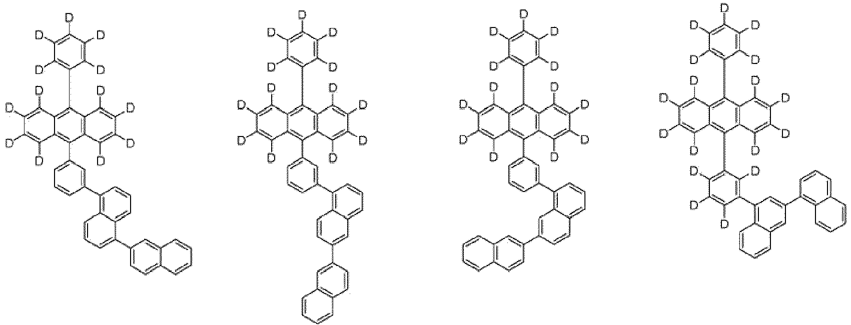

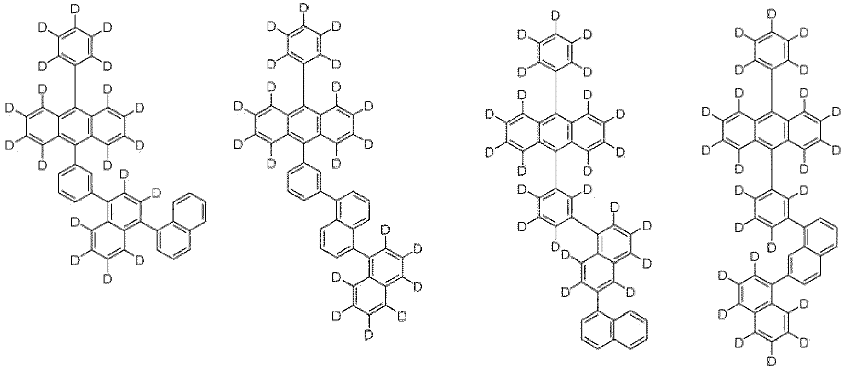

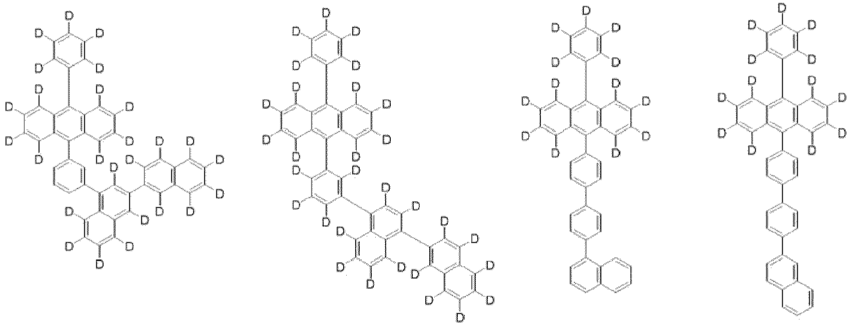

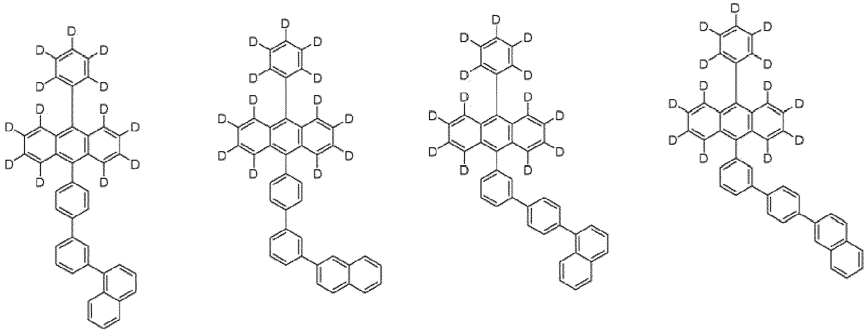

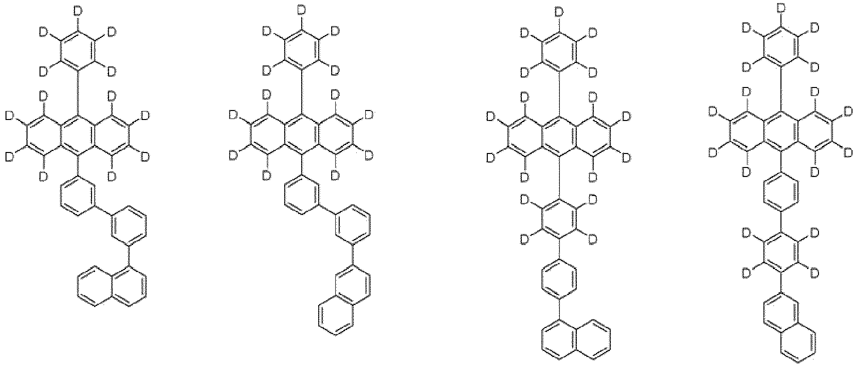

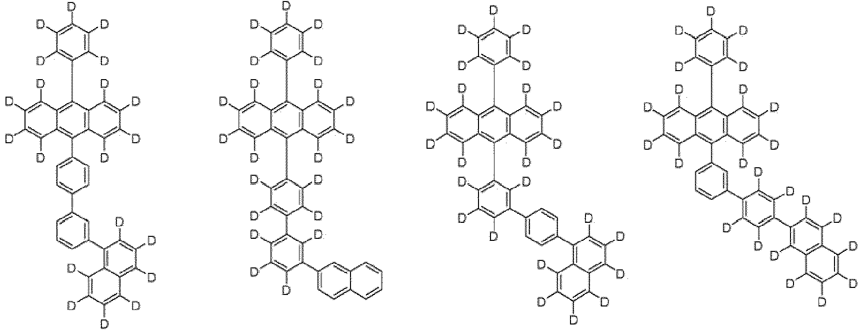

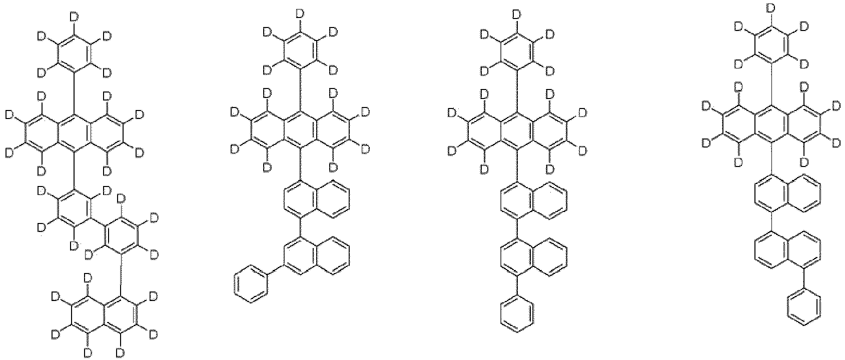

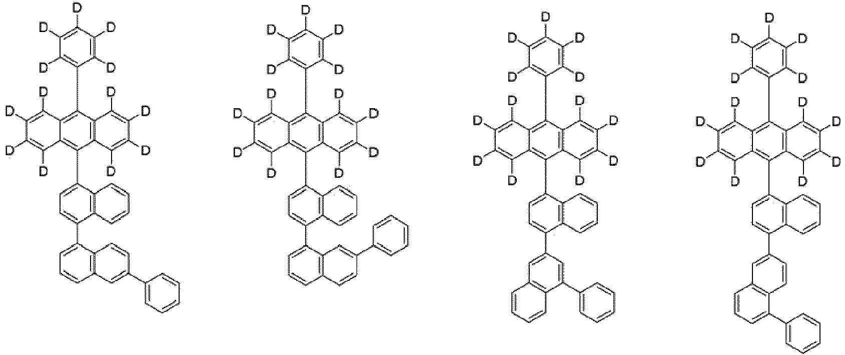

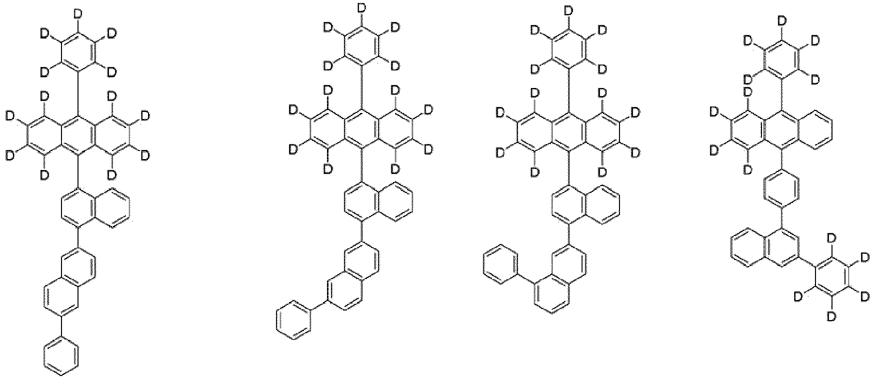

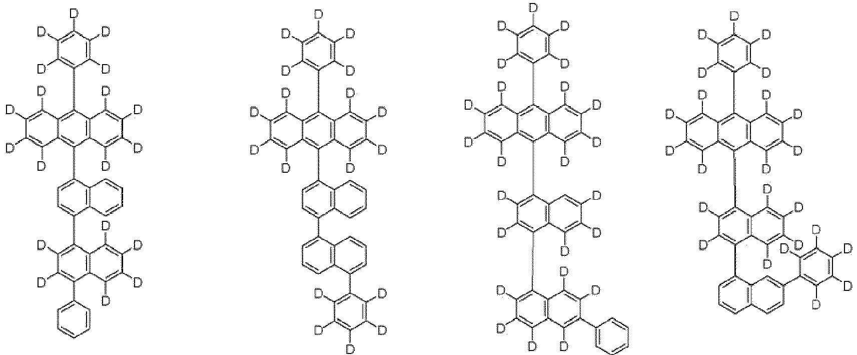

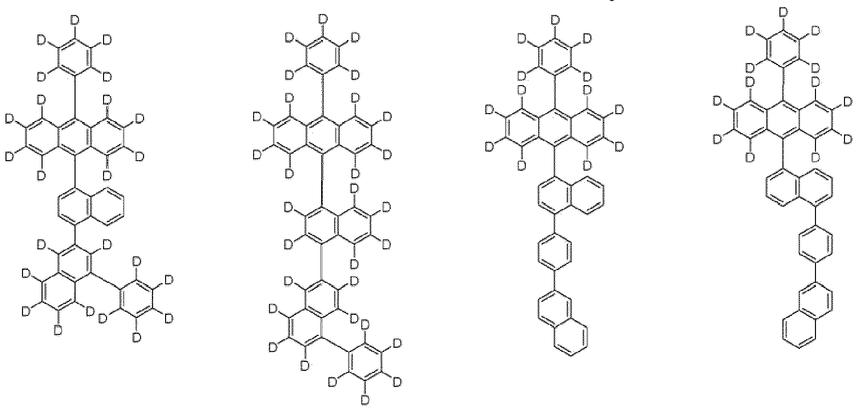

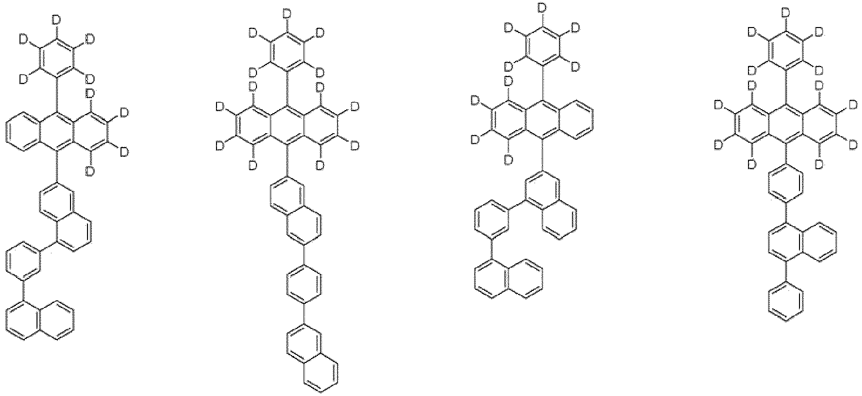

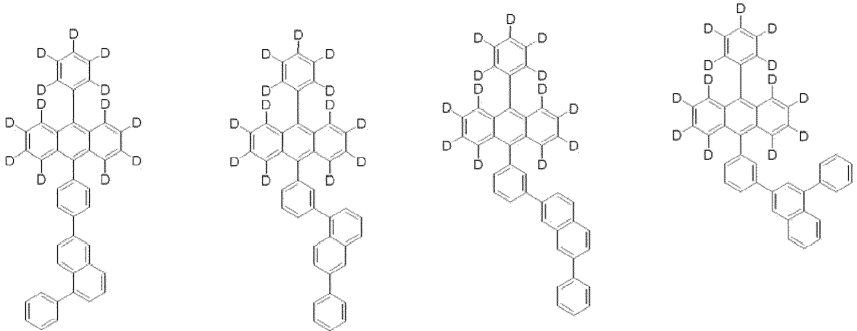

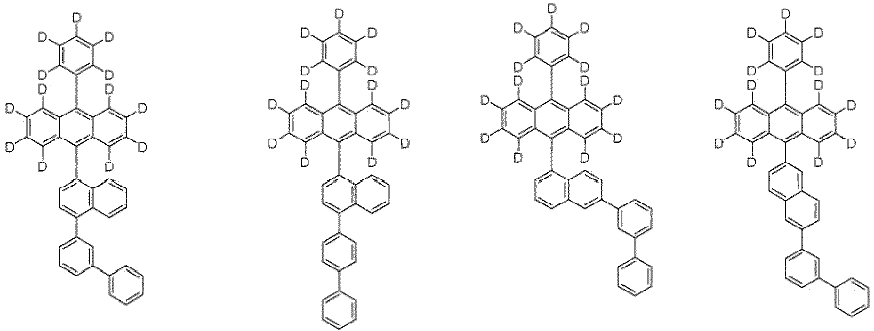

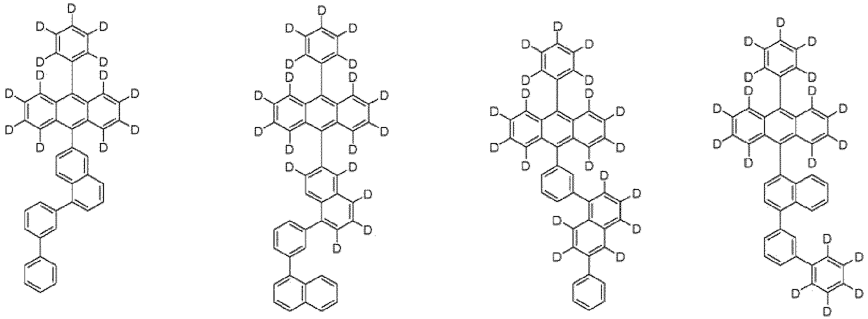

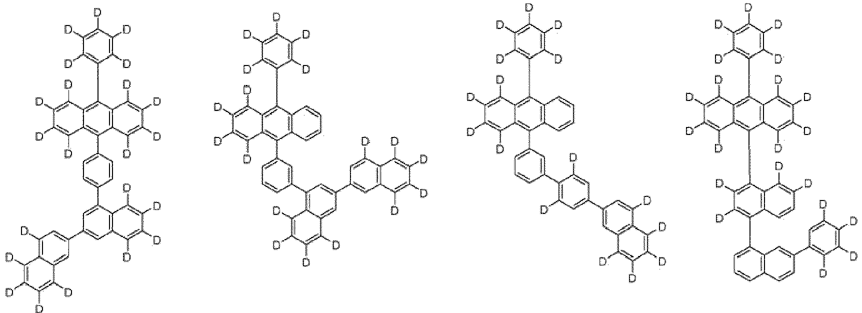

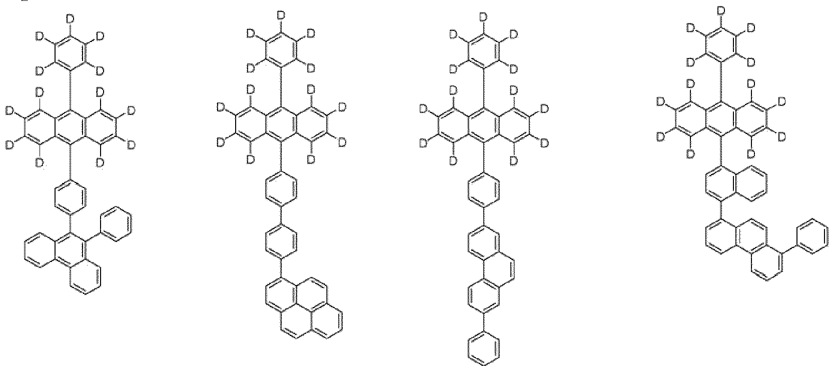

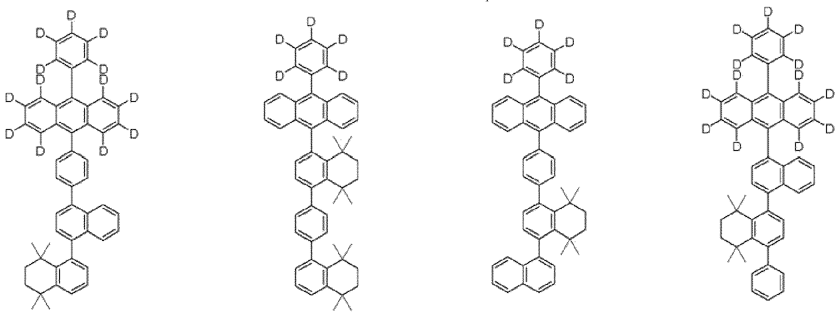

However, these compounds are not intended to limit the scope of Formula A.

According to one embodiment of the present invention, the anthracene derivative represented by Formula B may be selected from the following compounds:

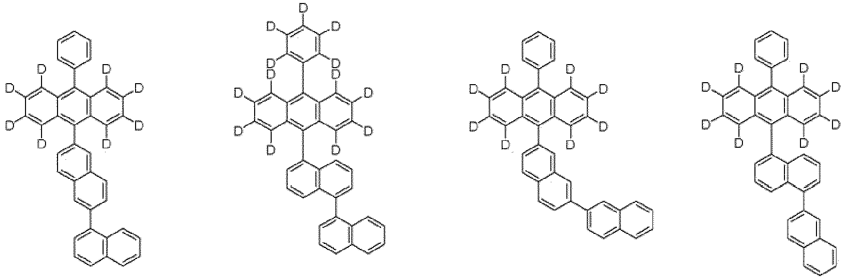

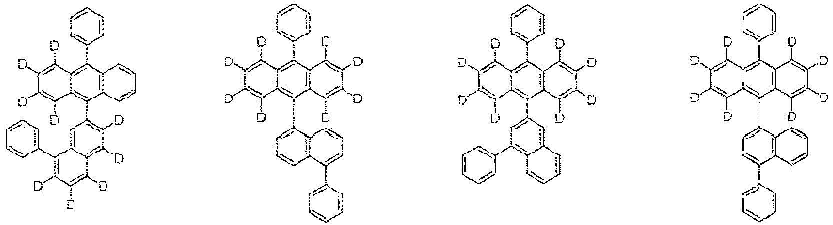

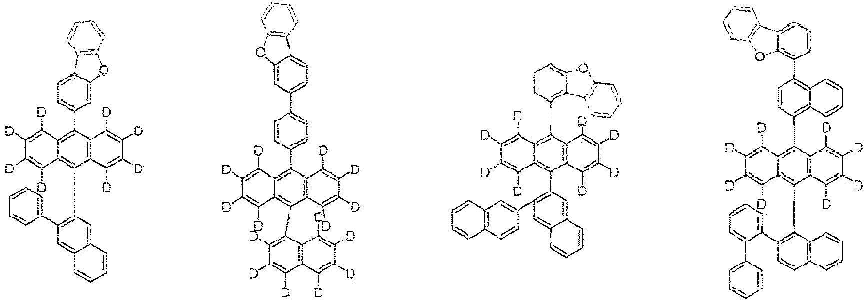

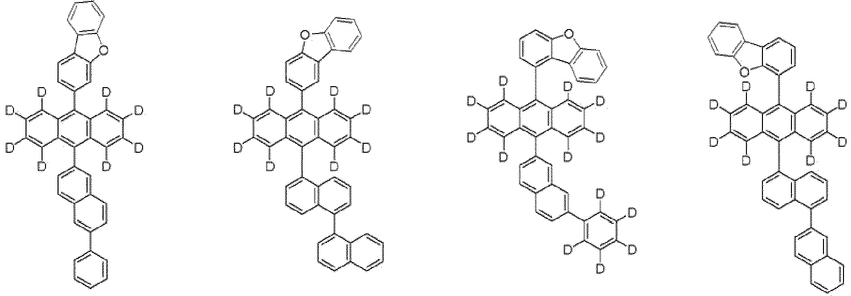

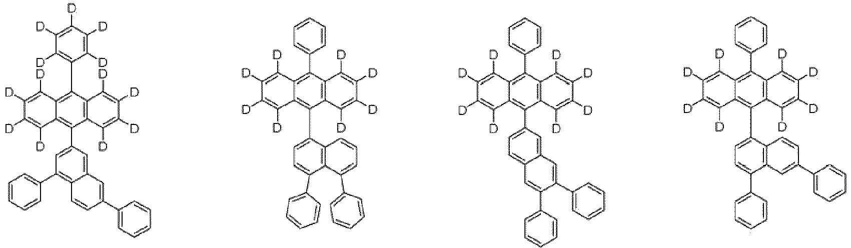

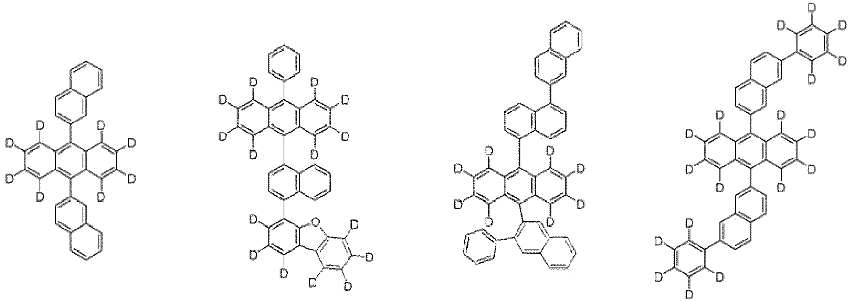

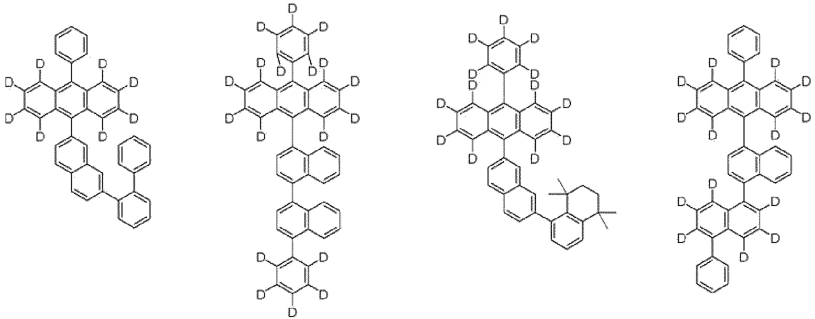

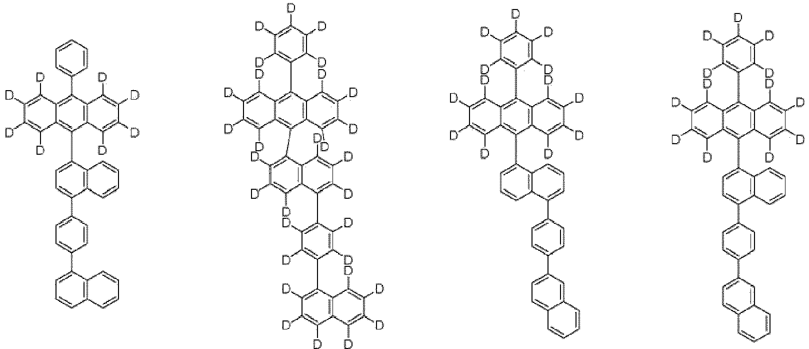

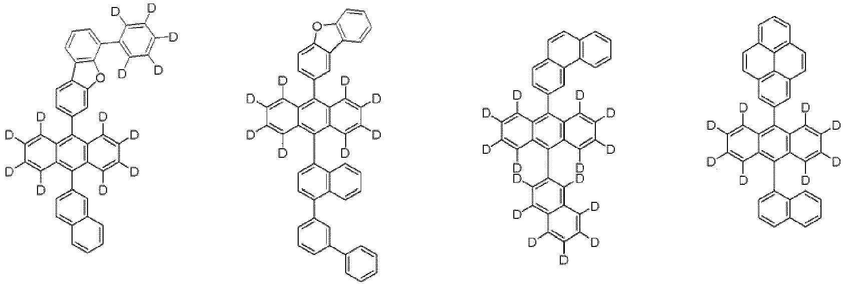

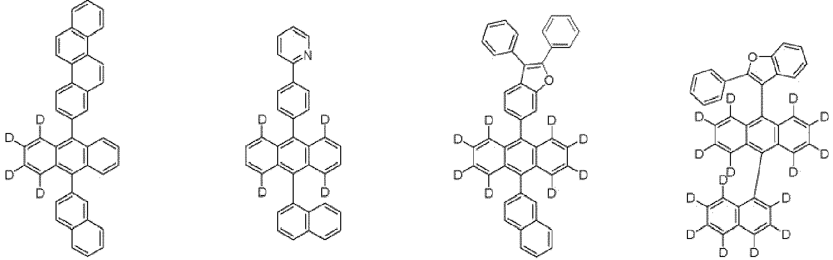

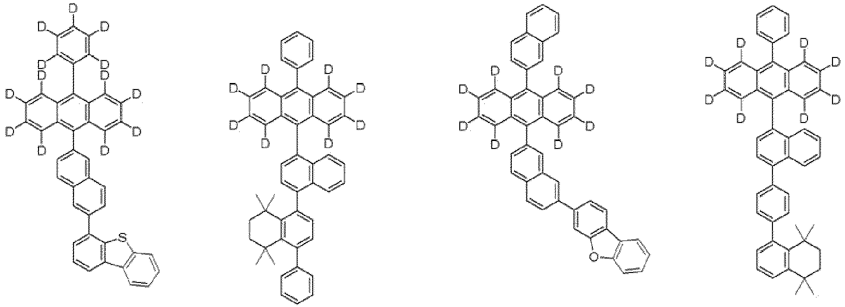

However, these compounds are not intended to limit the scope of Formula B.

According to one embodiment of the present invention, the polycyclic compound represented by Formula 1 may be selected from the following compounds:

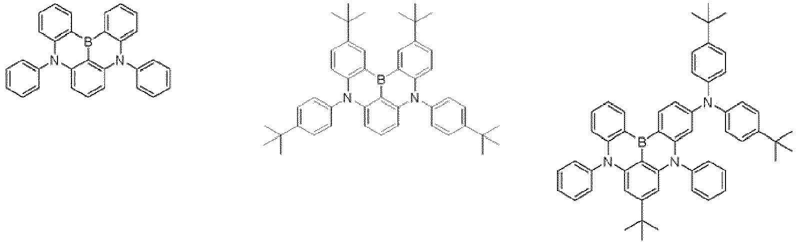

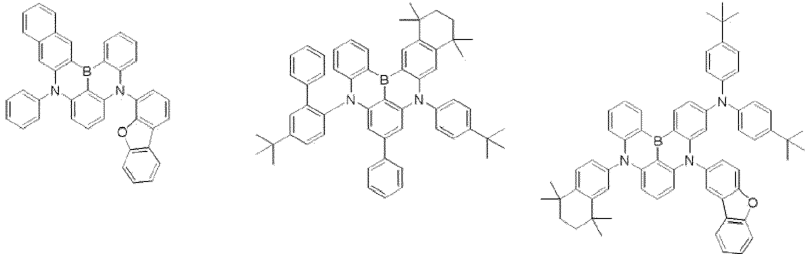

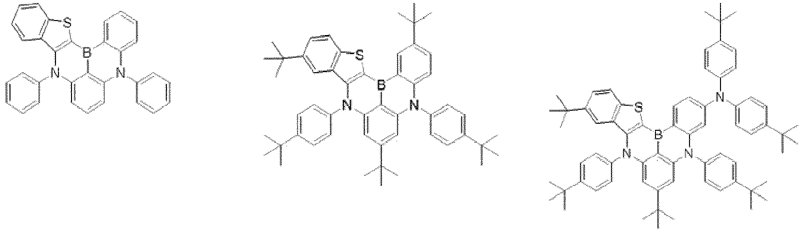

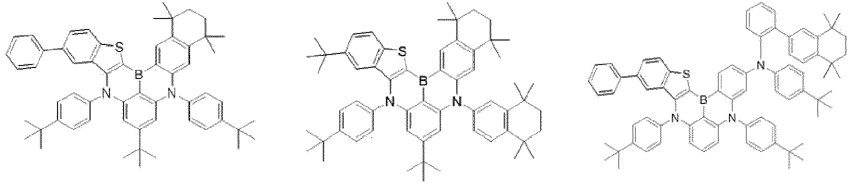

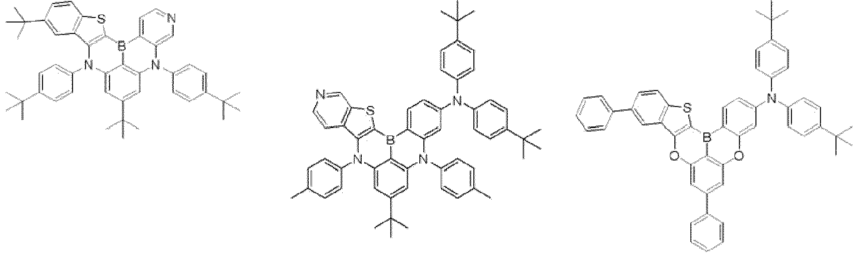

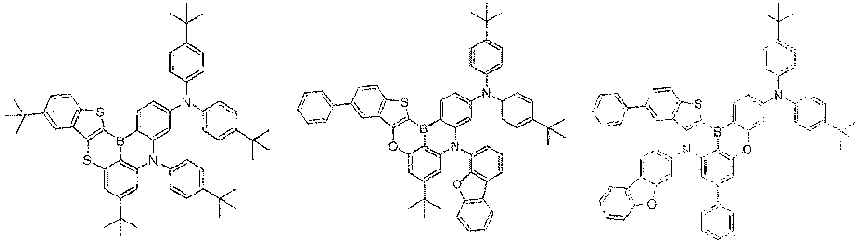

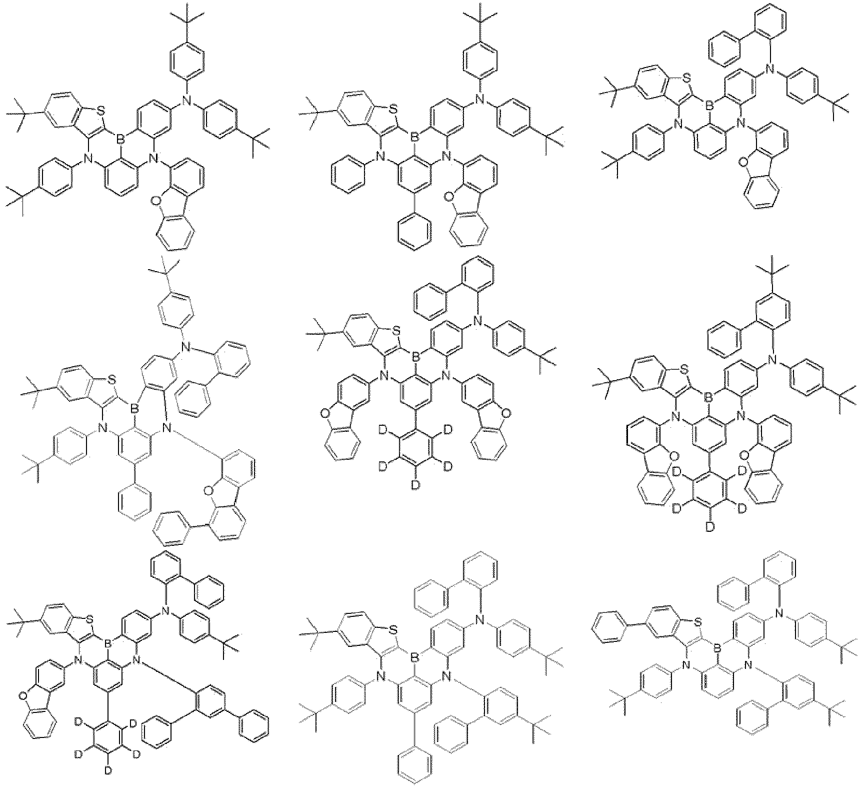

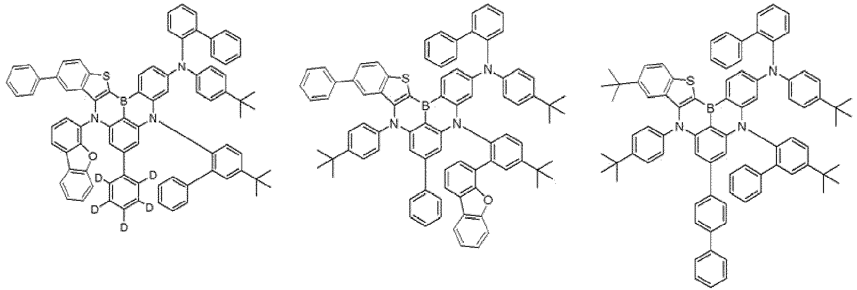

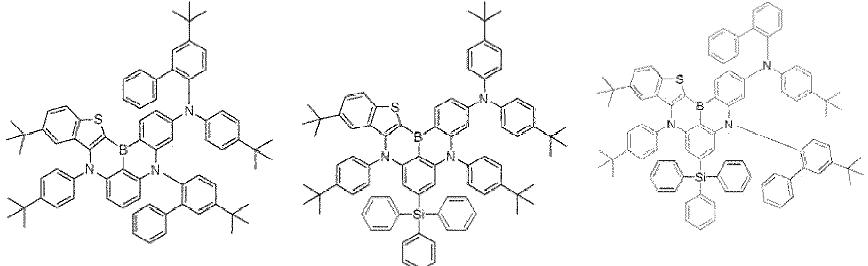

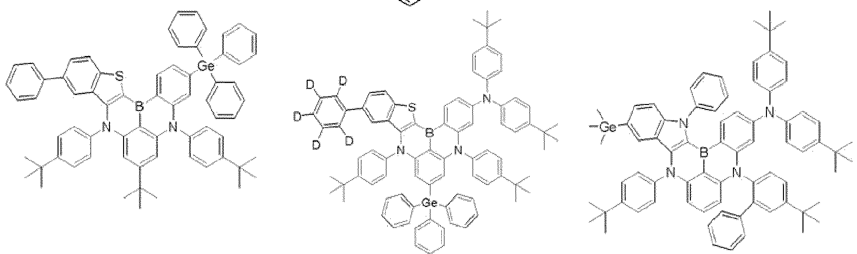

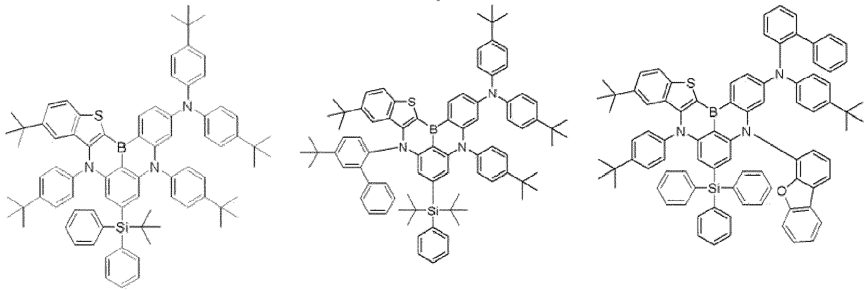

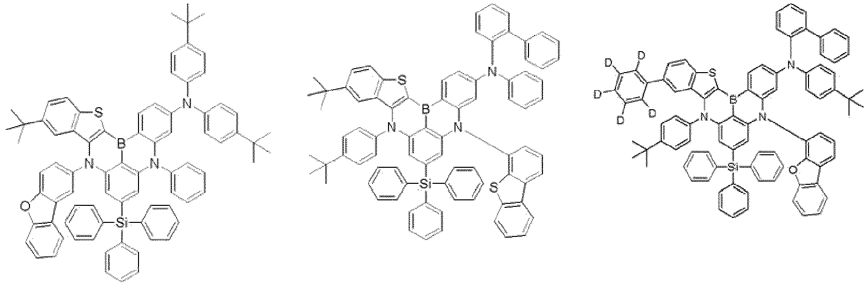

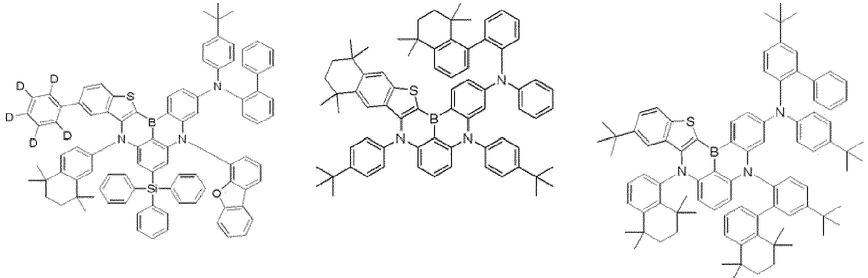

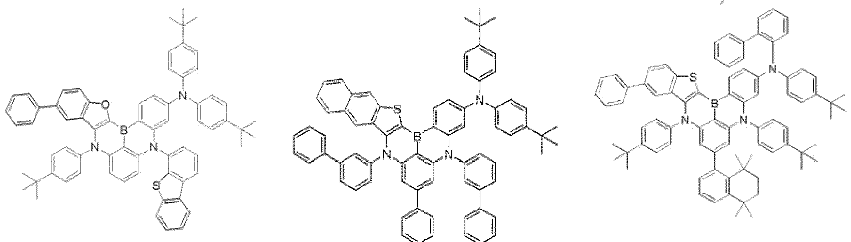

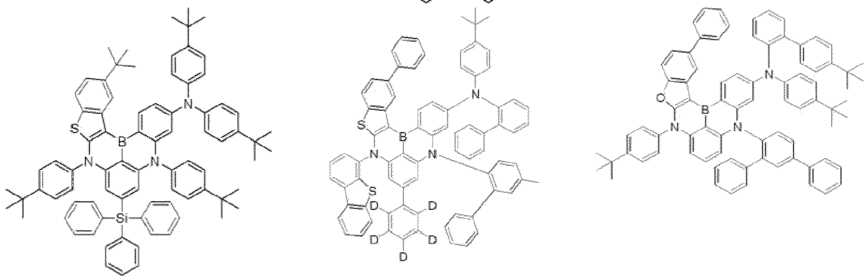

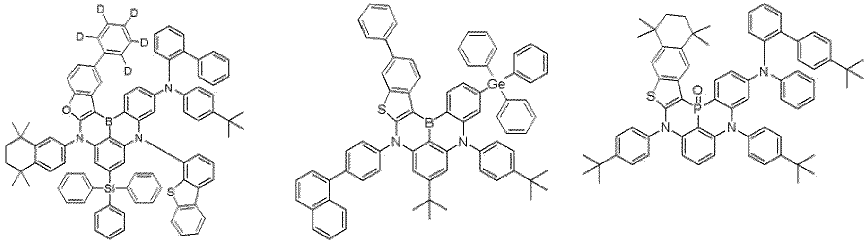

However, these compounds are not intended to limit the scope of Formula 1.

The organic layers of the organic light emitting device according to the present invention may form a monolayer structure. Alternatively, the organic layers may be stacked together to form a multilayer structure. For example, the organic layers may have a structure including a hole injecting layer, a hole transport layer, a hole blocking layer, a light emitting layer, an electron blocking layer, an electron transport layer, and an electron injecting layer but are not limited to this structure. The number of the organic layers is not limited and may be increased or decreased. Preferred structures of the organic layers of the organic light emitting device according to the present invention will be explained in more detail in the Examples section that follows.

A more detailed description will be given concerning exemplary embodiments of the organic light emitting device according to the present invention.

The organic light emitting device of the present invention includes an anode, a hole transport layer, a light emitting layer, an electron transport layer, and a cathode. The organic light emitting device of the present invention may optionally further include a hole injecting layer between the anode and the hole transport layer and an electron injecting layer between the electron transport layer and the cathode. If necessary, the organic light emitting device of the present invention may further include one or two intermediate layers such as a hole blocking layer or an electron blocking layer. The organic light emitting device of the present invention may further include one or more organic layers such as a capping layer that have various functions depending on the desired characteristics of the device.

A specific structure of the organic light emitting device according to one embodiment of the present invention, a method for fabricating the device, and materials for the organic layers are as follows.

First, an anode material is coated on a substrate to form an anode. The substrate may be any of those used in general organic light emitting devices. The substrate is preferably an organic substrate or a transparent plastic substrate that is excellent in transparency, surface smoothness, ease of handling, and waterproofness. A highly transparent and conductive metal oxide such as indium tin oxide (ITO), indium zinc oxide (IZO), tin oxide (SnO₂) or zinc oxide (ZnO) is used as the anode material.

A hole injecting material is coated on the anode by vacuum thermal evaporation or spin coating to form a hole injecting layer. Then, a hole transport material is coated on the hole injecting layer by vacuum thermal evaporation or spin coating to form a hole transport layer.

The hole injecting material is not specially limited so long as it is usually used in the art. Specific examples of such materials include 4,4',4"-tris(2-naphthylphenyl-phenylamino)triphenylamine (2-TNATA), N,N'-di(1-naphthyl)-N,N'-diphenylbenzidine (NPD), N,N'-diphenyl-N,N'-bis(3-methylphenyl)-1,1'-biphenyl-4,4'-diamine (TPD), and N,N'-diphenyl-N,N'-bis(4-(phenyl-m-tolylamino)phenyl)biphenyl-4,4'-diamine (DNTPD).

The hole transport material is not specially limited so long as it is commonly used in the art. Examples of such materials include N,N'-bis(3-methylphenyl)-N,N'-diphenyl-(1,1-biphenyl)-4,4'-diamine (TPD) and N,N'-di(naphthalen-1-yl)-N,N'-diphenylbenzidine (α-NPD).

Subsequently, a hole auxiliary layer and a light emitting layer are sequentially stacked on the hole transport layer. A hole blocking layer may be optionally formed on the light emitting layer by vacuum thermal evaporation or spin coating. The hole blocking layer is formed as a thin film and blocks holes from entering a cathode through the organic light emitting layer. This role of the hole blocking layer prevents the lifetime and efficiency of the device from deteriorating. A material having a very low highest occupied molecular orbital (HOMO) energy level is used for the hole blocking layer. The hole blocking material is not particularly limited so long as it can transport electrons and has a higher ionization potential than the light emitting compound. Representative examples of suitable hole blocking materials include BAlq, BCP, and TPBI.

Examples of materials for the hole blocking layer include, but are not limited to, BAlq, BCP, Bphen, TPBI, TAZ, BeBq₂, OXD-7, and Liq.

An electron transport layer is deposited on the hole blocking layer by vacuum thermal evaporation or spin coating, and an electron injecting layer is formed thereon. A cathode metal is deposited on the electron injecting layer by vacuum thermal evaporation to form a cathode, completing the fabrication of the organic light emitting device.

For example, lithium (Li), magnesium (Mg), aluminum (Al), aluminum-lithium (Al-Li), calcium (Ca), magnesium-indium (Mg-In) or magnesium-silver (Mg-Ag) may be used as the metal for the formation of the cathode. The organic light emitting device may be of top emission type. In this case, a transmissive material such as ITO or IZO may be used to form the cathode.

A material for the electron transport layer functions to stably transport electrons injected from the cathode. The electron transport material may be any of those known in the art and examples thereof include, but are not limited to, quinoline derivatives, particularly tris(8-quinolinolato)aluminum (Alq3), TAZ, BAlq, beryllium bis(benzoquinolin-10-olate) (Bebq2), and oxadiazole derivatives such as PBD, BMD, and BND.

Each of the organic layers can be formed by a monomolecular deposition or solution process. According to the monomolecular deposition process, the material for each layer is evaporated into a thin film under heat and vacuum or reduced pressure. According to the solution process, the material for each layer is mixed with a suitable solvent and the mixture is then formed into a thin film by a suitable method such as inkjet printing, roll-to-roll coating, screen printing, spray coating, dip coating or spin coating.

The organic light emitting device of the present invention can be used in a display or lighting system selected from flat panel displays, flexible displays, monochromatic flat panel lighting systems, white flat panel lighting systems, flexible monochromatic lighting systems, flexible white lighting systems, displays for automotive applications, displays for virtual reality, and displays for augmented reality.

### Mode for Carrying out the Invention

The present invention will be more specifically explained with reference to the following synthesis examples and fabrication examples. However, these examples are provided to assist in understanding the invention and are not intended to limit the scope of the present invention.

### Synthesis Example 1. Synthesis of BH-1

### Synthesis Example 1-1: Synthesis of A-1

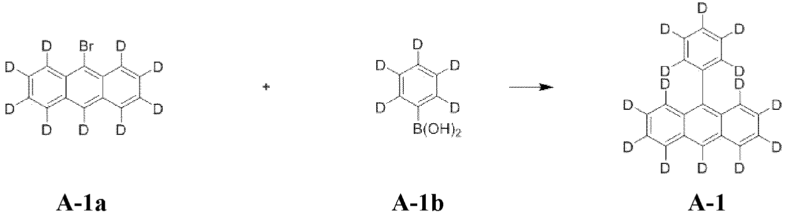

100 g of **A-1a,** 57.2 g of **A-1b,** 8.7 g of tetrakis(triphenylphosphine)palladium, and 103.8 g of potassium carbonate were placed in a 2 L round bottom flask as a reactor, and then 600 mL of toluene, 300 mL of ethanol, and 300 mL of water were added thereto. The temperature of the reactor was raised, and the mixture was stirred under reflux overnight. After completion of the reaction, the temperature of the reactor was lowered to room temperature. The reaction mixture was extracted with ethyl acetate. The organic layer was separated, concentrated under reduced pressure, and purified by column chromatography to afford **A-1** (80 g, 79.3%).

### Synthesis Example 1-2: Synthesis of A-2

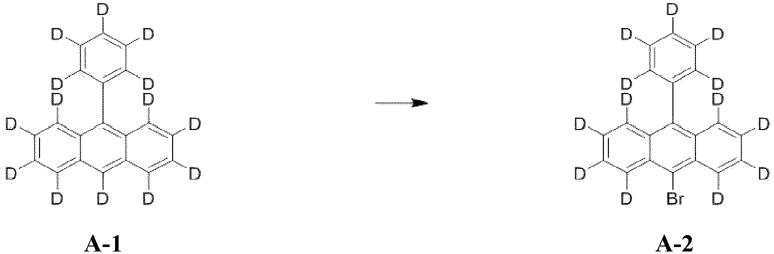

80 g of **A-1** was dissolved in 960 mL of dichloromethane in a 2 L round bottom flask as a reactor. The solution was cooled to 0 °C under a nitrogen atmosphere and a solution of 63.7 g of N-bromosuccinimide in N,N-dimethylformamide (200 mL) was slowly added dropwise thereto. After completion of the addition, the mixture was stirred at room temperature for 5 h. The completion of the reaction was confirmed by thin layer chromatography. Thereafter, the organic layer was washed with an aqueous sodium bicarbonate solution and washed once more with water. The organic layer was separated, filtered through a pad of silica gel, and concentrated under reduced pressure. The concentrate was recrystallized from dichloromethane and methanol to afford **A-2** (78 g, 75.6%).

### Synthesis Example 1-3: Synthesis of A-3

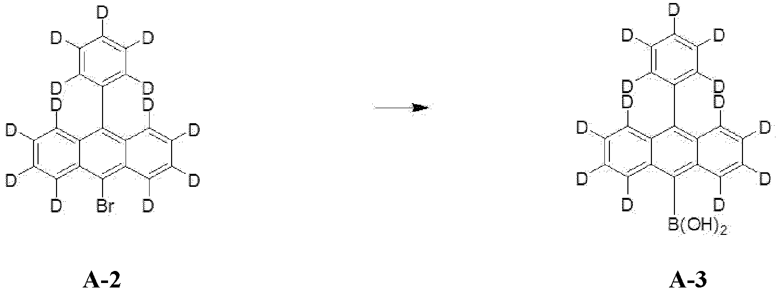

78 g of **A-2** was dissolved in 780 mL of tetrahydrofuran in a 2 L round bottom flask. The solution was cooled to -78 °C under a nitrogen atmosphere, followed by stirring. To the cooled solution was slowly added dropwise 162 mL of n-butyllithium. The mixture was stirred at the same temperature for 2 h, and then 29 g of trimethyl borate was slowly added dropwise thereto for 30 min. The resulting mixture was stirred at room temperature overnight. After completion of the reaction, the reaction mixture was acidified by slow dropwise addition of 2 N hydrochloric acid and extracted with water and ethyl acetate. The organic layer was separated and dried over magnesium sulfate. The residue was concentrated under reduced pressure and crystallized from heptane. The resulting solid was collected by filtration and washed with heptane and toluene to afford **A-3** (50 g, 71%).

### Synthesis Example 1-4: Synthesis of A-4

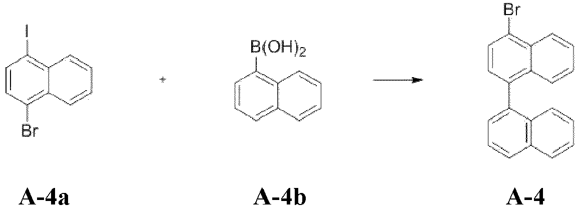

50 g of **A-4a,** 25.8 g of **A-4b,** 3.47 g of tetrakis(triphenylphosphine)palladium, and 41.5 g of potassium carbonate were placed in a 1 L round bottom flask as a reactor, and 300 mL of toluene, 150 mL of ethanol, and 150 mL of water were added thereto. The temperature of the reactor was raised, and the mixture was stirred under reflux overnight. After completion of the reaction, the temperature of the reactor was lowered to room temperature. The reaction mixture was extracted with ethyl acetate. The organic layer was separated, concentrated under reduced pressure, and purified by column chromatography to afford **A-4** (42 g, 83.9%).

### Synthesis Example 1-5: Synthesis of A-5

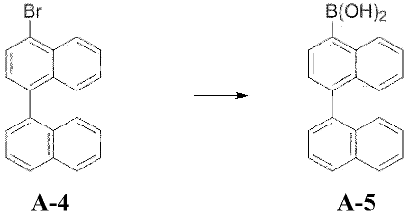

**A-5** (yield 83.8%) was synthesized in the same manner as in Synthesis Example 1-3, except that **A-4** was used instead of **A-2**.

### Synthesis Example 1-6: Synthesis of A-6

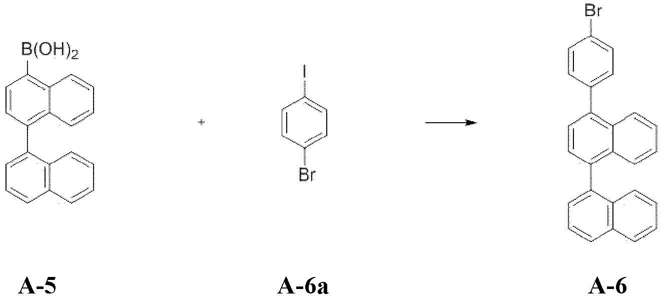

29.9 g of **A-6a,** 31.5 g of **A-5**, 2.44 g of tetrakis(triphenylphosphine)palladium, and 29.2 g of potassium carbonate were placed in a 1 L round bottom flask as a reactor, and 180 mL of toluene, 90 mL of ethanol, and 90 mL of water were added thereto. The temperature of the reactor was raised, and the mixture was stirred under reflux overnight. After completion of the reaction, the temperature of the reactor was lowered to room temperature. The reaction mixture was extracted with ethyl acetate. The organic layer was separated, concentrated under reduced pressure, and purified by column chromatography to afford **A-6** (43.9 g, 91%).

### Synthesis Example 1-7: Synthesis of BH-1

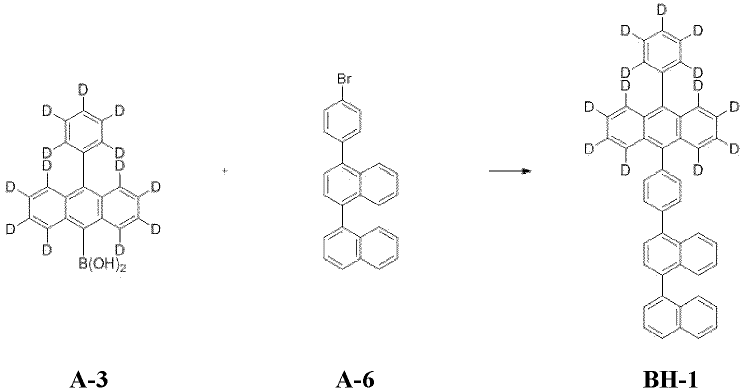

13.7 g of **A-3**, 15 g of **A-6**, 0.85 g of tetrakis(triphenylphosphine)palladium, and 10.1 g of potassium carbonate were placed in a 0.5 L round bottom flask as a reactor, and 90 mL of toluene, 45 mL of ethanol, and 45 mL of water were added thereto. The temperature of the reactor was raised, and the mixture was stirred under reflux overnight. The temperature of the reactor was further increased, and the resulting mixture was further stirred under reflux for 4 h. After completion of the reaction, the temperature of the reactor was lowered to room temperature. Ethanol was added to precipitate a crystal, followed by filtration. The solid was dissolved in toluene, filtered through silica gel, and concentrated under reduced pressure. The solid was recrystallized from toluene and acetone to afford **BH-1** (12.2 g, 55.9%).
MS (MALDI-TOF): m/z 595.32 [M⁺]

### Synthesis Example 2. Synthesis of BH-2

### Synthesis Example 2-1: Synthesis of B-1

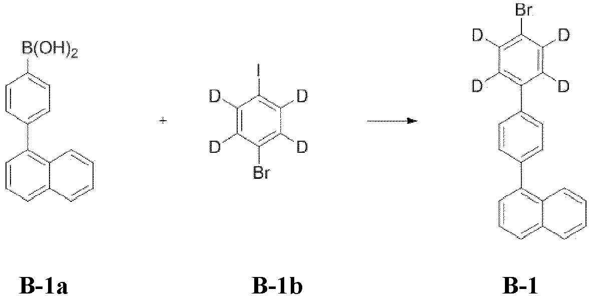

**B-1** (yield 76.2%) was synthesized in the same manner as in Synthesis Example 1-1, except that **B-1b** and **B-1a** were used instead of **A-1a** and **A-1b,** respectively.

### Synthesis Example 2-2: Synthesis of BH-2

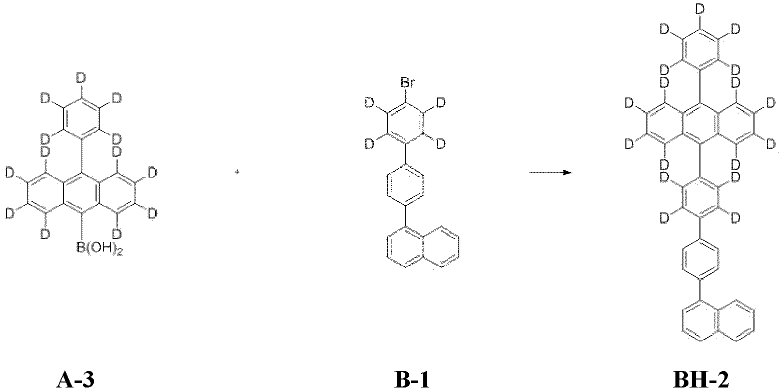

**BH-2** (yield 50.7%) was synthesized in the same manner as in Synthesis Example 1-7, except that **B-1** was used instead of **A-6.**
MS (MALDI-TOF): m/z 549.33 [M⁺]

### Synthesis Example 3. Synthesis of BH-3

### Synthesis Example 3-1: Synthesis of C-1

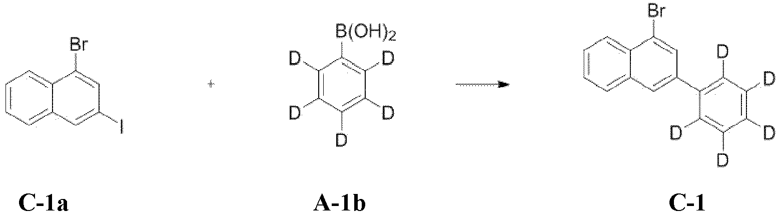

**C-1** (yield 87.8%) was synthesized in the same manner as in Synthesis Example 1-1, except that **C-1a** was used instead of **A-1a**.

### Synthesis Example 3-2: Synthesis of C-2

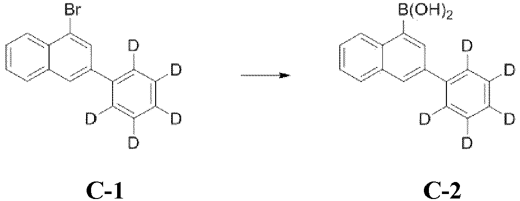

**C-2** (yield 84.4%) was synthesized in the same manner as in Synthesis Example 1-3, except that C-1 was used instead of **A-2**.

### Synthesis Example 3-3: Synthesis of C-3

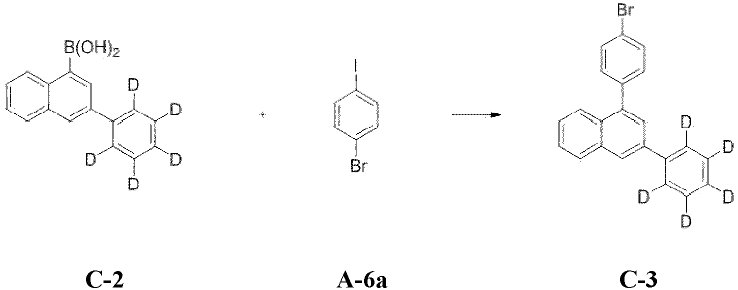

**C-3** (yield 86.8%) was synthesized in the same manner as in Synthesis Example 1-1, except that **A-6a** and **C-2** were used instead of **A-1a** and **A-1b,** respectively.

### Synthesis Example 3-4: Synthesis of C-4

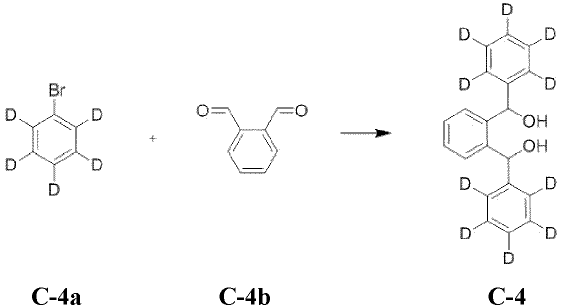

79.7 g of **C-4a** and 797 mL of tetrahydrofuran were placed in a 2 L round bottom flask and the inside of the reactor was cooled to -78 °C under a nitrogen atmosphere. The mixture was stirred and 307.5 mL of n-butyllithium was slowly added dropwise thereto. To the resulting mixture was added portionwise 30 g of phthalaldehyde. Thereafter, the mixture was heated to room temperature, followed by stirring for 2 h. The reaction was quenched by addition of 500 mL of water. The reaction mixture was extracted with ethyl acetate and water. The organic layer was separated and concentrated under reduced pressure to afford **C-4** (52 g, 77.4%).

### Synthetic Example 3-5: Synthesis of C-5

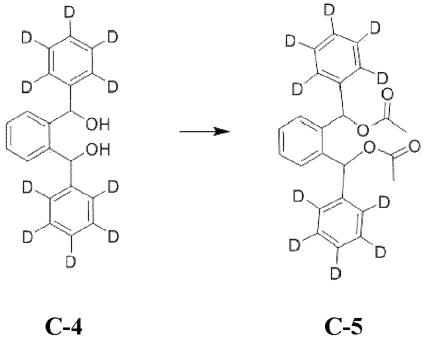

52 g of **C-4,** 520 mL of dichloromethane, 70.7 g of acetic anhydride, and 105.1 g of triethylamine were placed in a 2 L round bottom flask. The mixture was stirred at 0 °C. 4.23 g of 4-dimethylaminopyridine was added portionwise to the reactor. The resulting mixture was heated to room temperature, followed by stirring. After completion of the reaction, the reaction mixture was added with 500 mL of water, stirred, and extracted. The organic layer was separated and concentrated under reduced pressure to afford **C-5** (61.5 g, 92.4%).

### Synthesis Example 3-6: Synthesis of C-6

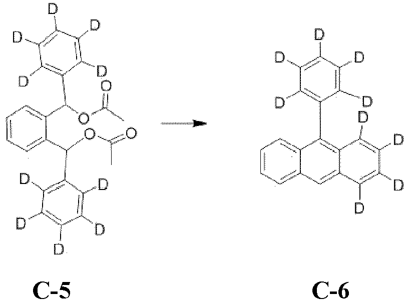

61.5 g of **C-5** and 615 mL of dichloromethane were stirred in a 2 L round bottom flask. To the mixture was added 1.7 mL of sulfuric acid. After completion of the reaction, the reaction mixture was added with 600 mL of water, stirred, and extracted. The organic layer was separated, concentrated under reduced pressure, and added with excess methanol. The resulting solid was collected by filtration to afford **C-6** (37.4 g, 88.8%).

### Synthesis Example 3-7: Synthesis of C-7

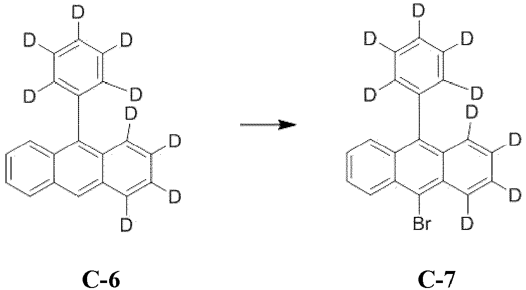

C-7 (yield 72.4%) was synthesized in the same manner as in Synthesis Example 1-2, except that **C-6** was used instead of **A-1.**

### Synthesis Example 3-8: Synthesis of C-8

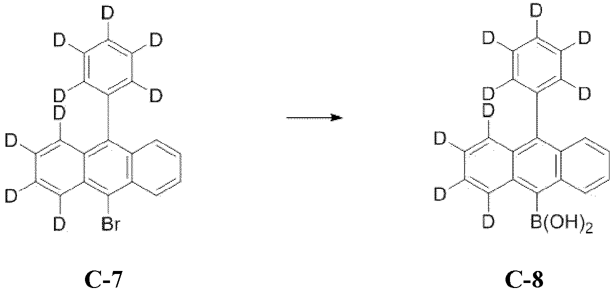

**C-8** (yield 89.3%) was synthesized in the same manner as in Synthesis Example 1-3, except that **C-7** was used instead of **A-2**.

### Synthesis Example 3-9: Synthesis of BH-3

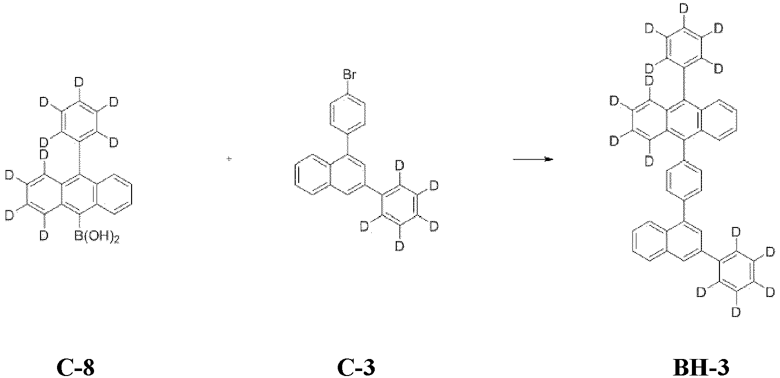

**BH-3** (yield 56.9%) was synthesized in the same manner as in Synthesis Example 1-7, except that **C-8** and **C-3** were used instead of **A-3** and **A-6**, respectively.
MS (MALDI-TOF): m/z 546.31 [M⁺]

### Synthesis Example 4. Synthesis of BH-4

### Synthesis Example 4-1: Synthesis of D-1

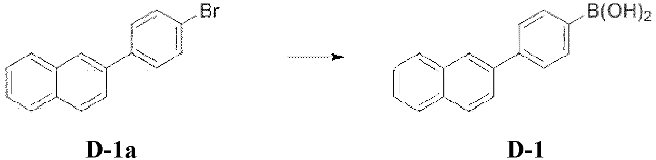

**D-1** (yield 82.6%) was synthesized in the same manner as in Synthesis Example 1-3, except that **D-1a** was used instead of **A-2**.

### Synthesis Example 4-2: Synthesis of D-2

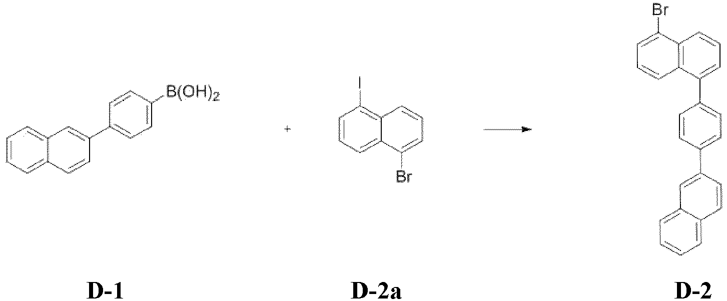

**D-2** (yield 85.3%) was synthesized in the same manner as in Synthesis Example 1-1, except that D-2a and D-1 were used instead of **A-1a** and **A-1b,** respectively.

### Synthesis Example 4-3: Synthesis of BH-4

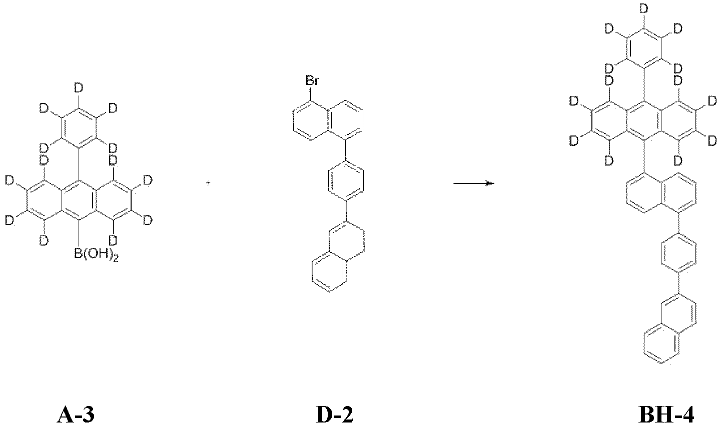

**BH-4** (yield 60.5%) was synthesized in the same manner as in Synthesis Example 1-7, except that D-2 was used instead of **A-6.**
MS (MALDI-TOF): m/z 595.32 [M⁺]

### Synthesis Example 5. Synthesis of BH-5

### Synthesis Example 5-1: Synthesis of E-1

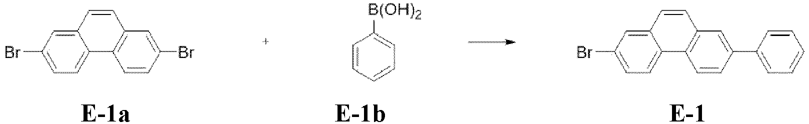

**E-1** (yield 66.8%) was synthesized in the same manner as in Synthesis Example 1-1, except that **E-1a** and **E-1b** were used instead of **A-1a** and **A-1b,** respectively.

### Synthesis Example 5-2: Synthesis of E-2

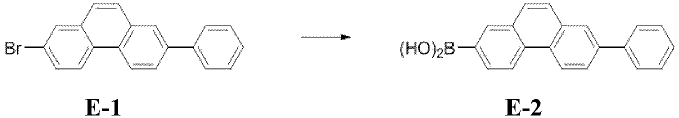

**E-2** (yield 88.8%) was synthesized in the same manner as in Synthesis Example 1-3, except that **E-1** was used instead of **A-2**.

### Synthesis Example 5-3: Synthesis of E-3

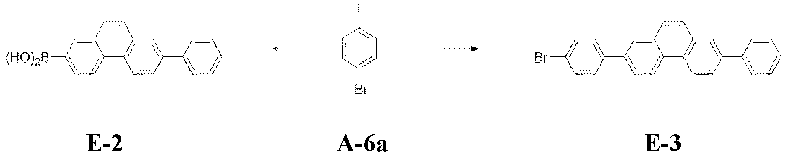

**E-3** (yield 78.2%) was synthesized in the same manner as in Synthesis Example 1-1, except that **A-6a** and **E-2** were used instead of **A-1a** and **A-1b,** respectively.

### Synthesis Example 5-4: Synthesis of BH-5

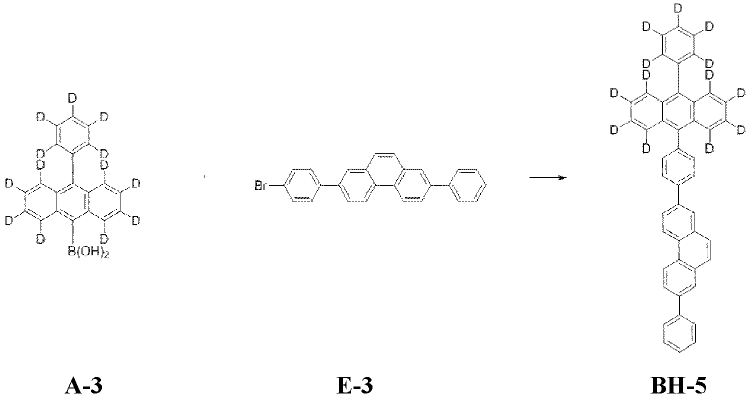

**BH-5** (yield 53.6%) was synthesized in the same manner as in Synthesis Example 1-7, except that **E-3** was used instead of **A-6.**
MS (MALDI-TOF): m/z 595.32 [M⁺]

### Synthesis Example 6. Synthesis of BH-6

### Synthesis Example 6-1: Synthesis of F-1

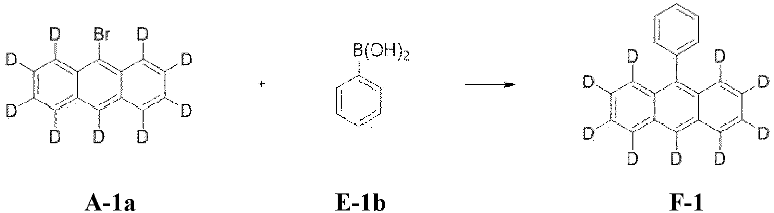

**F-1** (yield 94.2%) was synthesized in the same manner as in Synthesis Example 1-1, except that **E-1b** was used instead of **A-1b**.

### Synthesis Example 6-2: Synthesis of F-2

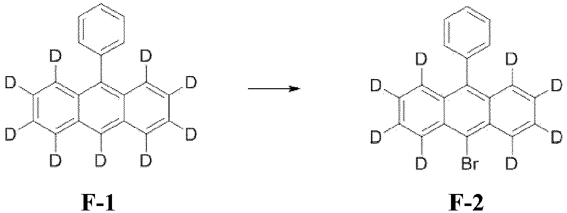

**F-2** (yield 91.4%) was synthesized in the same manner as in Synthesis Example 1-2, except that **F-1** was used instead of **A-1.**

### Synthesis Example 6-3: Synthesis of F-3

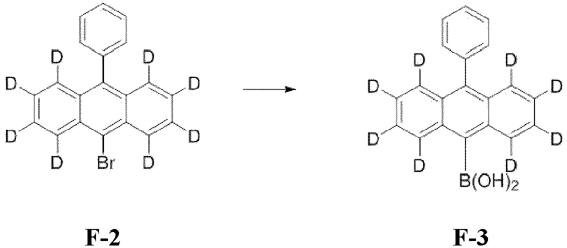

**F-3** (yield 85.8%) was synthesized in the same manner as in Synthesis Example 1-3, except that **F-2** was used instead of **A-2**.

### Synthesis Example 6-4: Synthesis of F-4

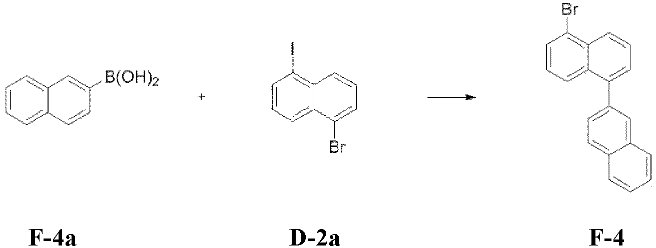

**F-4** (yield 88.3%) was synthesized in the same manner as in Synthesis Example 1-1, except that **D-2a** and **F-4a** were used instead of **A-1a** and **A-1b,** respectively.

### Synthesis Example 6-5: Synthesis of BH-6

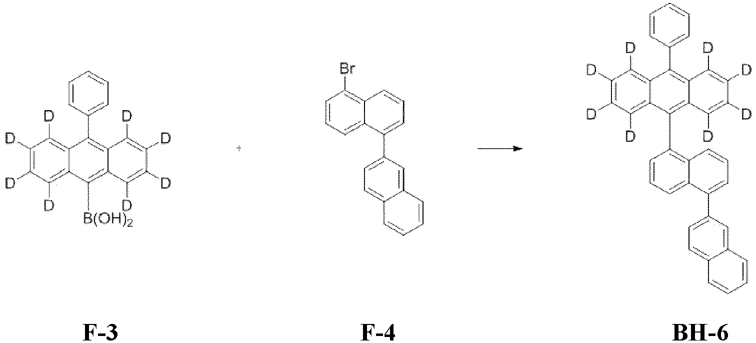

**BH-6** (yield 46.6%) was synthesized in the same manner as in Synthesis Example 1-7, except that **F-3** and **F-4** were used instead of **A-3** and **A-6**, respectively.
MS (MALDI-TOF): m/z 514.25 [M⁺]

### Synthesis Example 7. Synthesis of BH-7

### Synthesis Example 7-1: Synthesis of G-1

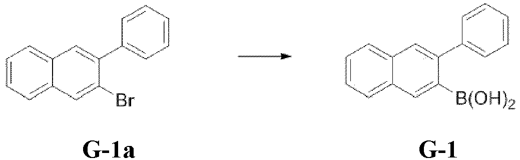

**G-1** (yield 78.8%) was synthesized in the same manner as in Synthesis Example 1-3, except that **G-1a** was used instead of **A-2**.

### Synthesis Example 7-2: Synthesis of G-2

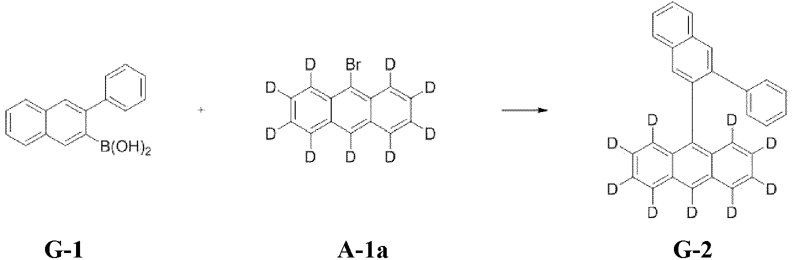

G-2 (yield 86.7%) was synthesized in the same manner as in Synthesis Example 1-1, except that **G-1** was used instead of **A-1b.**

### Synthesis Example 7-3: Synthesis of G-3

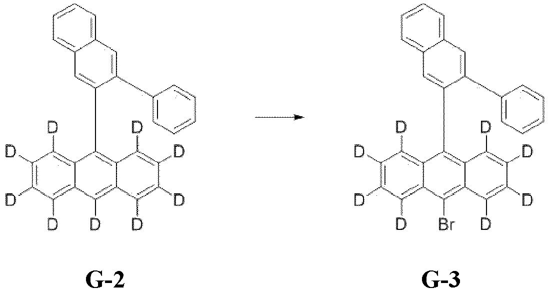

**G-3** (yield 84.8%) was synthesized in the same manner as in Synthesis Example 1-2, except that **G-2** was used instead of **A-1.**

### Synthesis Example 7-4: Synthesis of BH-7

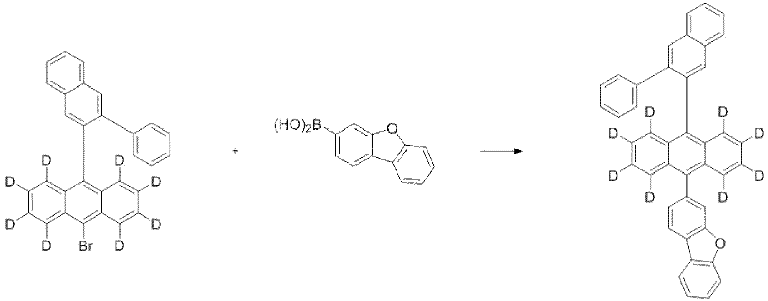

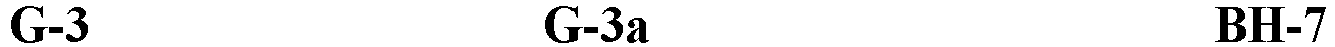

**BH-7** (yield 58.1%) was synthesized in the same manner as in Synthesis Example 1-7, except that **G-3a** and **G-3** were used instead of **A-3** and **A-6,** respectively.

### MS (MALDI-TOF): m/z 554.25 [M⁺]

### Examples 1-7: Fabrication of organic light emitting devices

ITO glass was patterned to have a light emitting area of 2 mm × 2 mm, followed by cleaning. After the cleaned ITO glass was mounted in a vacuum chamber, the base pressure was adjusted to 1 × 10⁻⁷ torr. TNATA (400 Å) and HT (200 Å) were deposited in this order on the ITO glass. The inventive host compound shown in Table 1 was mixed with 2 wt% of the inventive dopant compound BD-1 or BD-2. The mixture was used to form a 250 Å thick light emitting layer. Then, the compound of Formula E-1 and Liq were sequentially deposited to form a 300 Å thick electron transport layer and a 10 Å electron injecting layer on the light emitting layer, and Al (1,000 Å) was deposited thereon to fabricate an organic light emitting device. The luminescent properties of the organic light emitting device were measured at 10 mA/cm².

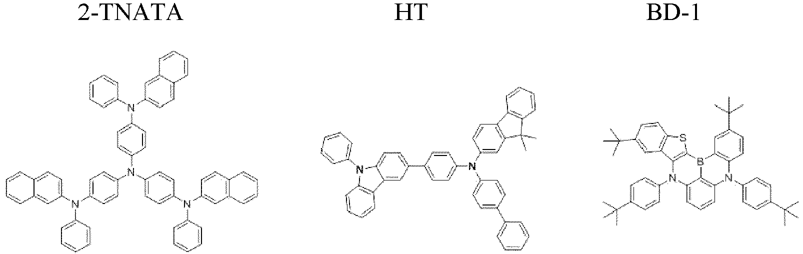

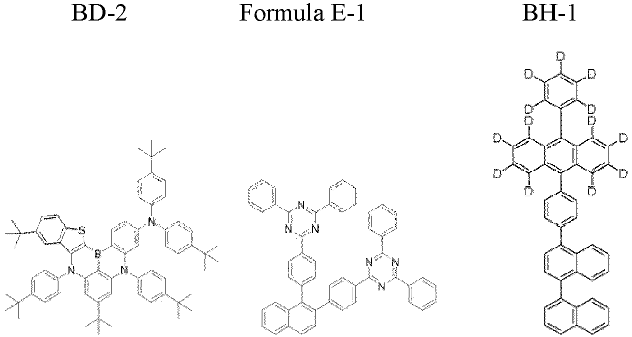

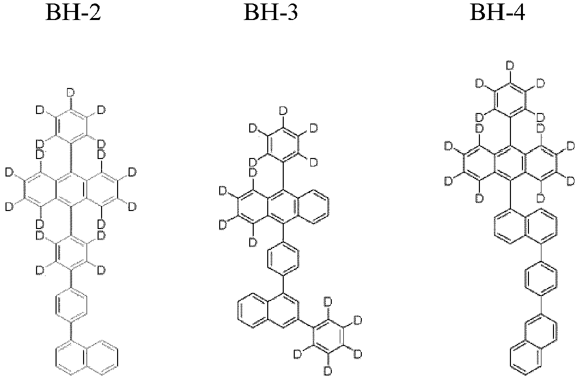

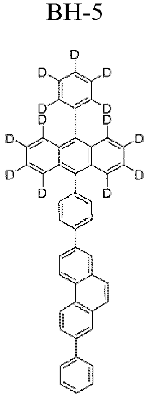

### Comparative Examples 1-4

Organic light emitting devices were fabricated in the same manner as in Examples 1-7, except that RH-1 or RH-2 was used as a host compound instead of the inventive compound. The luminescent properties of the organic light emitting devices were measured at 10 mA/cm². The structures of RH-1 and RH-2 are as follow:

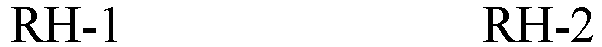

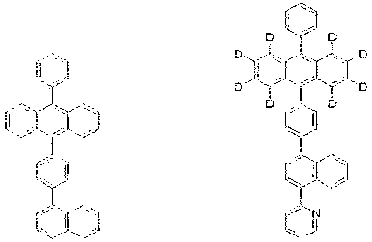

**Table 1**

| Example No. | Host | Dopant | External quantum efficiency (%) | Lifetime (T97, hr) |
|---|---|---|---|---|
| Example 1 | BH-1 | BD-1 | 8.1 | 146 |
| Example 2 | BH-2 | BD-1 | 8.0 | 135 |
| Example 3 | BH-3 | BD-1 | 8.2 | 123 |
| Example 4 | BH-4 | BD-1 | 8.4 | 151 |
| Example 5 | BH-5 | BD-1 | 8.0 | 140 |
| Example 6 | BH-3 | BD-2 | 9.3 | 135 |
| Example 7 | BH-4 | BD-2 | 9.8 | 166 |
| Comparative Example 1 | RH-1 | BD-1 | 7.1 | 91 |
| Comparative Example 2 | RH-2 | BD-1 | 7.2 | 54 |
| Comparative Example 3 | RH-1 | BD-2 | 7.7 | 100 |
| Comparative Example 4 | RH-2 | BD-2 | 7.7 | 67 |

As can be seen from the results in Table 1, the organic light emitting devices of Examples 1-7, each of which employed the inventive compound as a host material in the light emitting layer, had high external quantum efficiencies and improved life characteristics compared to the organic light emitting devices of Comparative Examples 1-4, each of which employed the conventional anthracene derivative whose specific structure is contrasted with that of the inventive compound.

### Examples 8-17: Fabrication of organic light emitting devices

Organic light emitting devices were fabricated in the same manner as in Examples 1-7, except that a mixture of the host compound and BH-6 or BH-7 in a ratio of 1:1 was used instead of the host compound alone and BD-3 was used instead of the dopant compound. The luminescent properties of the organic light emitting devices were measured at 10 mA/cm². The structures of BH-6, BH-7, and BD-3 are as follow:

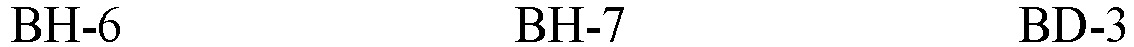

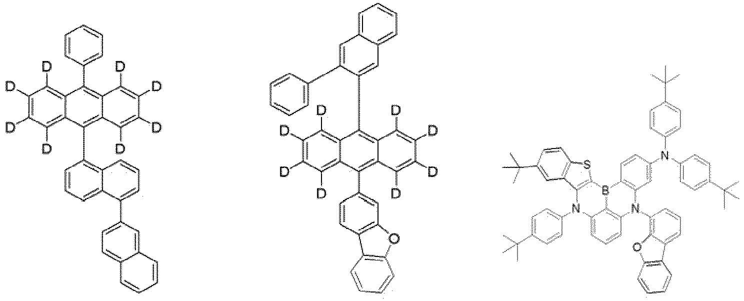

### Comparative Examples 5-9

Organic light emitting devices were fabricated in the same manner as in Examples 8-17, except that RH-3 or RH-4 was used instead of the inventive host compound. The luminescent properties of the organic light emitting devices were measured at 10 mA/cm². The structures of RH-3 and RH-4 are as follow:

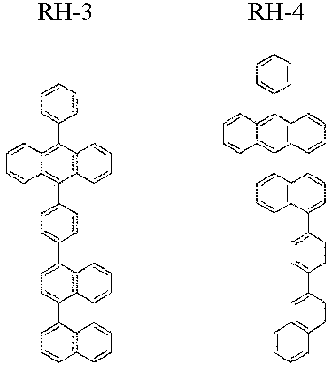

**Table 2**

| Example No. | Hosts (1:1) | External quantum efficiency (%) | Lifetime (T97, hr) |
|---|---|---|---|
| Example 8 | BH-1 : BH-6 | 9.5 | 152 |
| Example 9 | BH-2 : BH-6 | 9.3 | 145 |
| Example 10 | BH-3 : BH-6 | 9.9 | 142 |
| Example 11 | BH-4 : BH-6 | 10.2 | 164 |
| Example 12 | BH-5 : BH-6 | 9.2 | 157 |
| Example 13 | BH-1 : BH-7 | 9.9 | 158 |
| Example 14 | BH-2 : BH-7 | 9.8 | 150 |
| Example 15 | BH-3 : BH-7 | 102 | 147 |
| Example 16 | BH-4 : BH-7 | 106 | 155 |
| Example 17 | BH-5 : BH-7 | 9.5 | 163 |
| Comparative Example 5 | RH-2 : BH-6 | 8.1 | 76 |
| Comparative Example 6 | RH-2 : BH-7 | 8.1 | 85 |
| Comparative Example 7 | BH-1 : RH-3 | 8.3 | 114 |
| Comparative Example 8 | BH-4 : RH-4 | 8.7 | 127 |
| Comparative Example 9 | RH-2 : RH-4 | 7.9 | 71 |

As can be seen from the results in Table 2, the organic light emitting devices of Examples 8-17, each of which employed the inventive compounds as host materials in the light emitting layer, had high efficiencies and long lifetimes compared to the organic light emitting devices of Comparative Examples 5-9, each of which employed the conventional anthracene derivative whose specific structure is contrasted with that of the inventive compound instead of one of the host materials.

### Industrial Applicability

The organic light emitting device of the present invention includes a light emitting layer employing, as a host, the anthracene derivative having a specific structure in which an aryl moiety is introduced to a skeletal structure containing a deuterated phenyl moiety and an anthracene moiety linked to the deuterated phenyl moiety. The use of the host ensures high luminous efficiency and improved life characteristics of the device. Due to these advantages, the highly efficient and long-lasting organic light emitting device can find applications in not only lighting systems but also a variety of displays, including flat panel displays, flexible displays, and wearable displays.

## Claims

1. An anthracene derivative represented by Formula A: 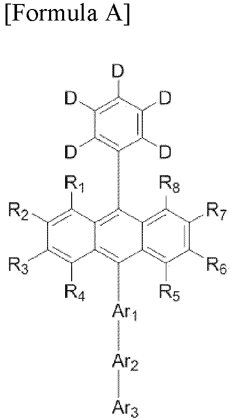 wherein R₁ to R₈ are identical to or different from each other and are each independently hydrogen or deuterium and Ar₁ to Ar₃ are identical to or different from each other and are each independently substituted or unsubstituted C₆-C₅₀ aryl or substituted or unsubstituted C₃-C₅₀ mixed aliphatic-aromatic cyclic group, the "substituted" in the definitions of Ar₁ to Ar₃ in Formula A indicating substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof, the "unsubstituted" in the same definition indicating having no substituent, and one or more hydrogen atoms in each of the substituents being optionally replaced by deuterium atoms.

2. The anthracene compound according to claim 1, wherein at least four of R₁ to R₈ are deuterium.

3. The anthracene compound according to claim 1, wherein each of Ar₁ to Ar₃ is independently substituted or unsubstituted C₆-C₂₄ aryl that are deuterated.

4. The anthracene compound according to claim 1, wherein each of Ar₁ to Ar₃ is selected from Structural Formulas 1 to 3:

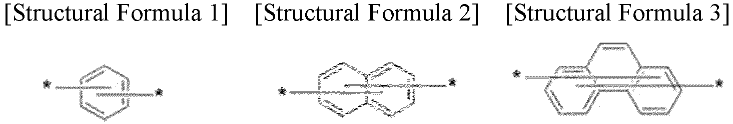

5. The anthracene compound according to claim 1, wherein the anthracene compound represented by Formula A is selected from the following compounds:

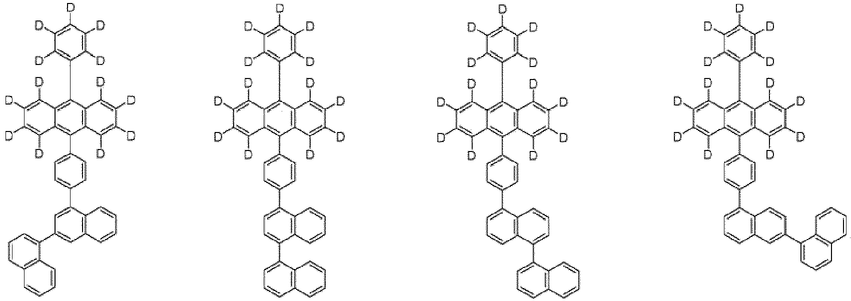

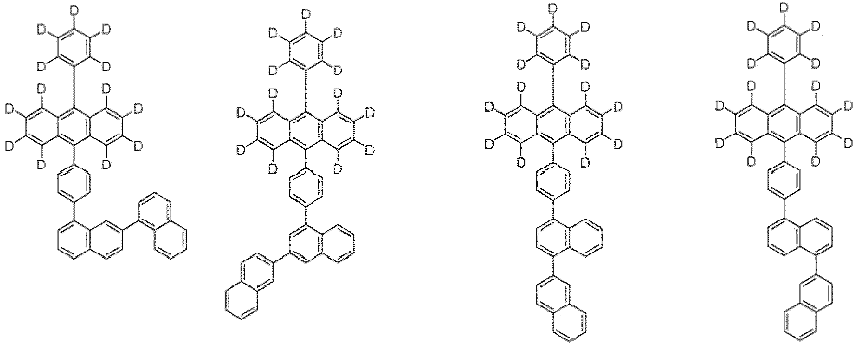

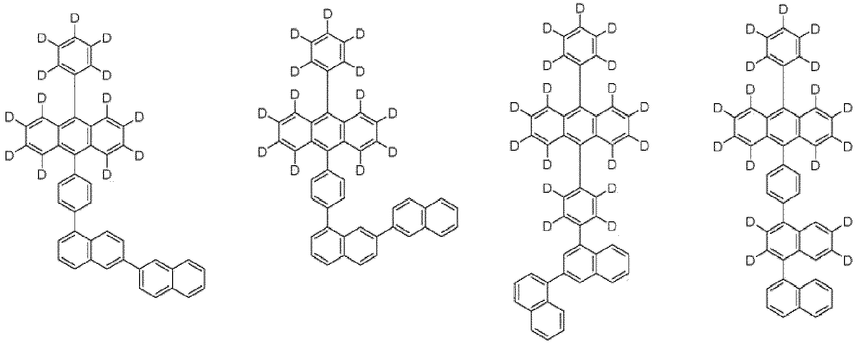

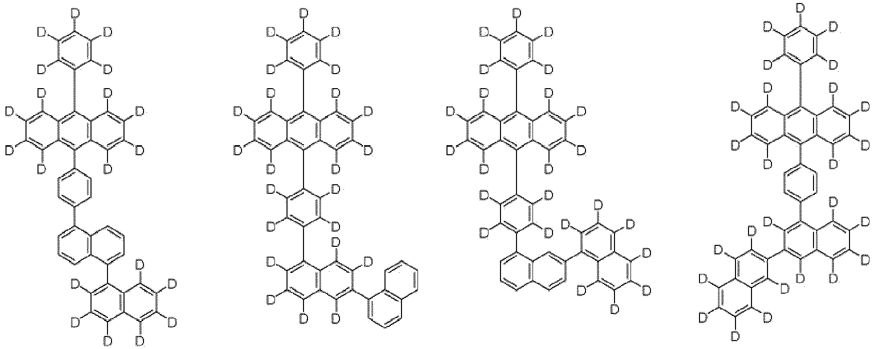

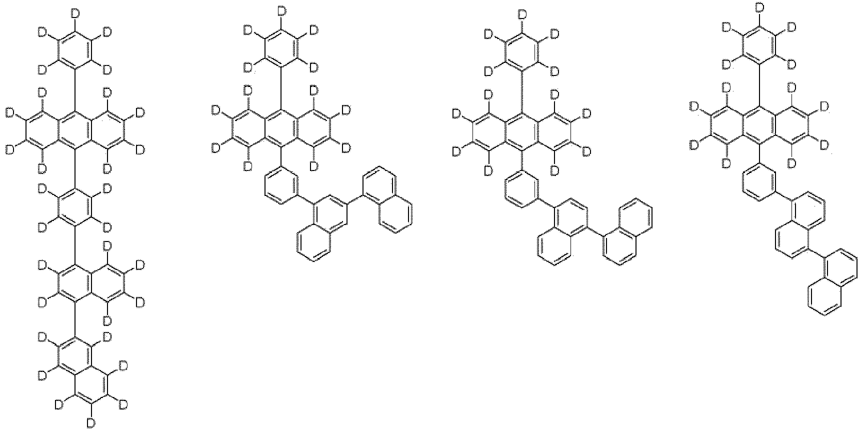

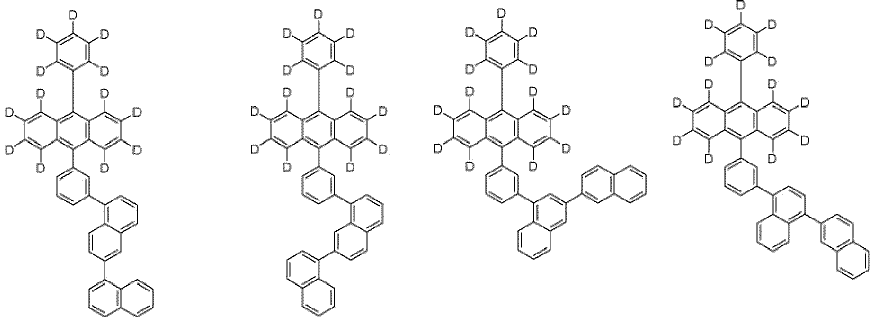

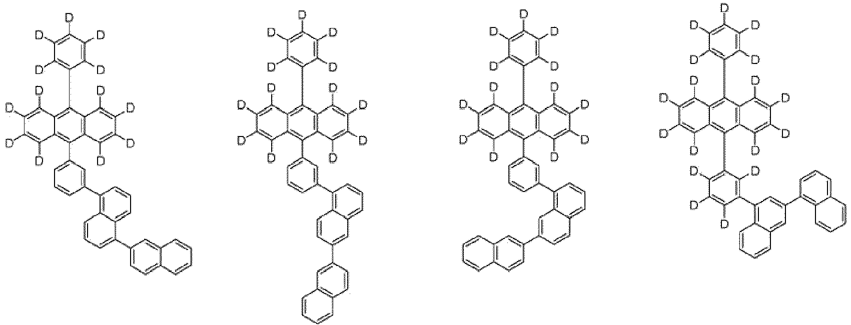

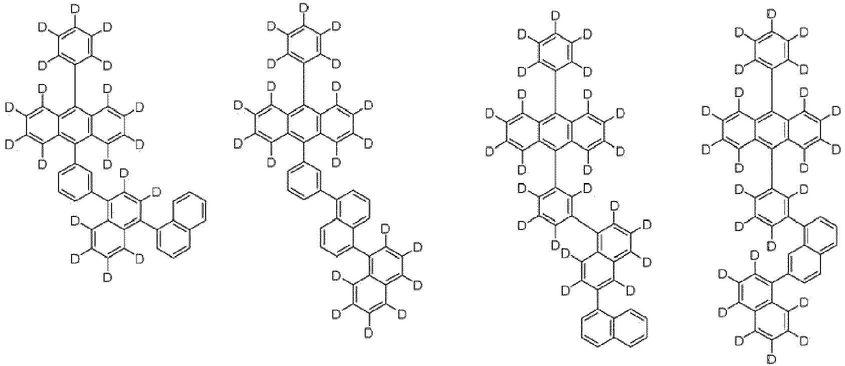

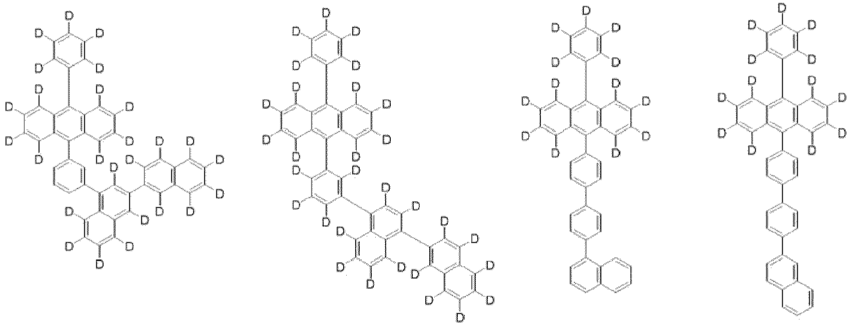

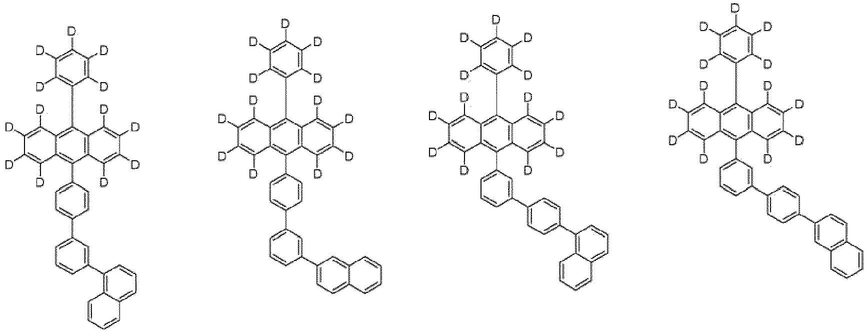

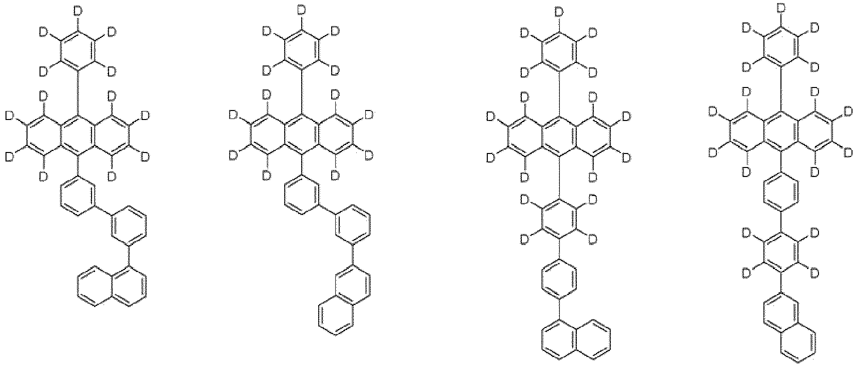

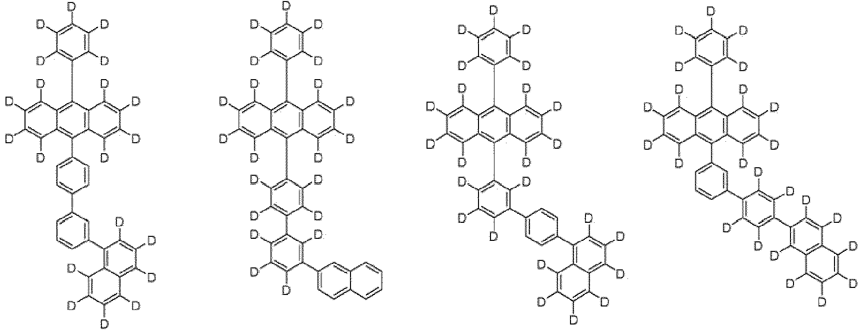

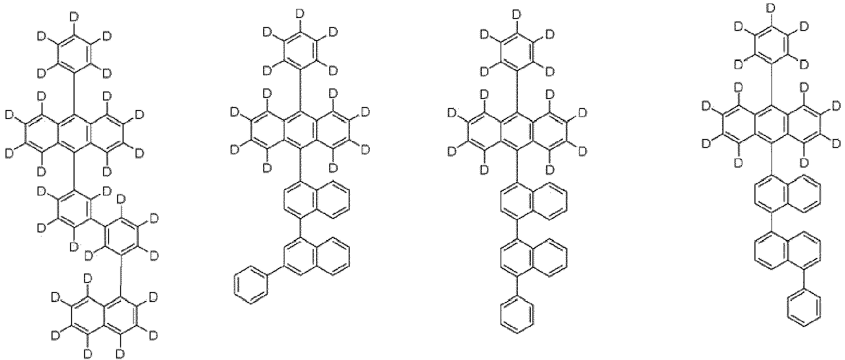

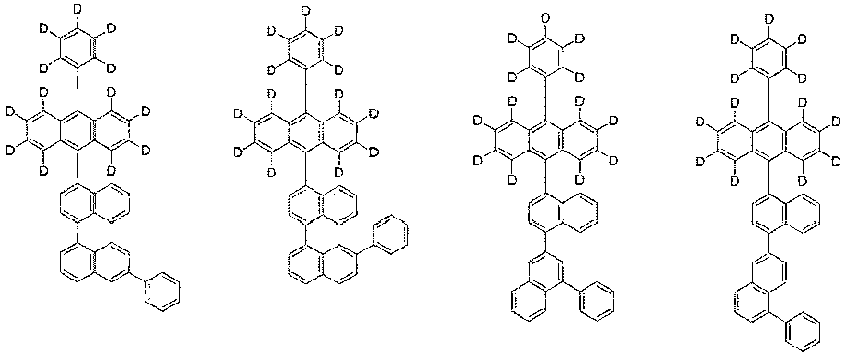

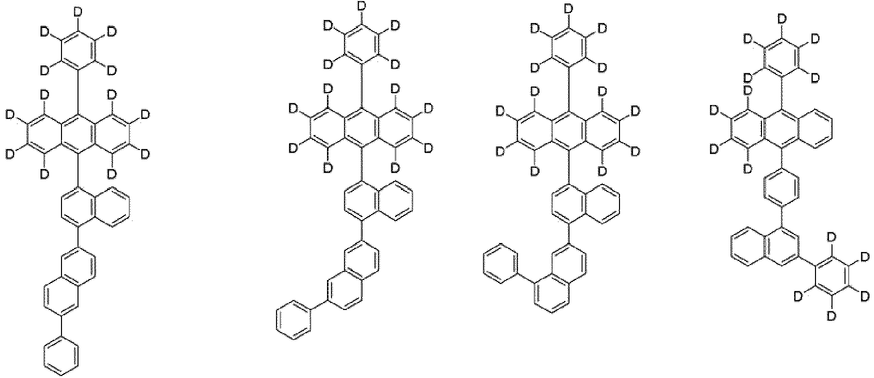

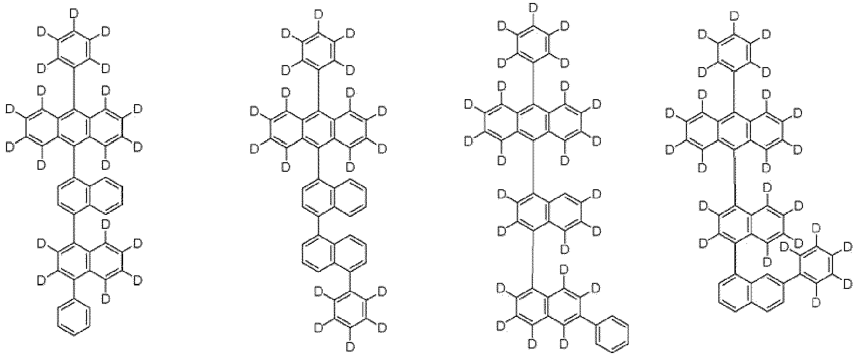

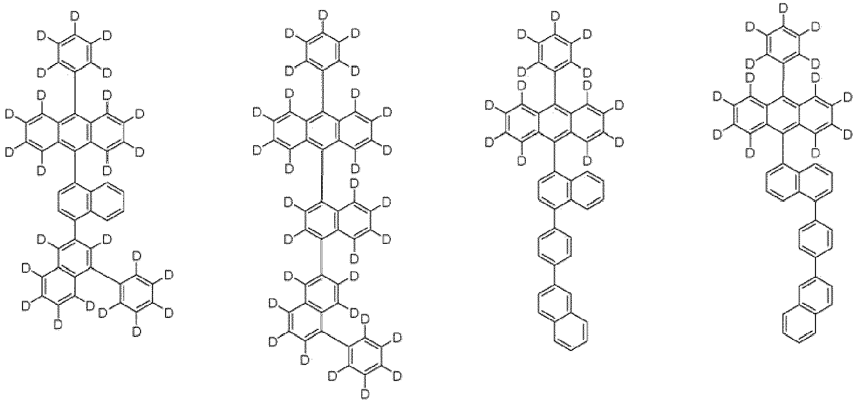

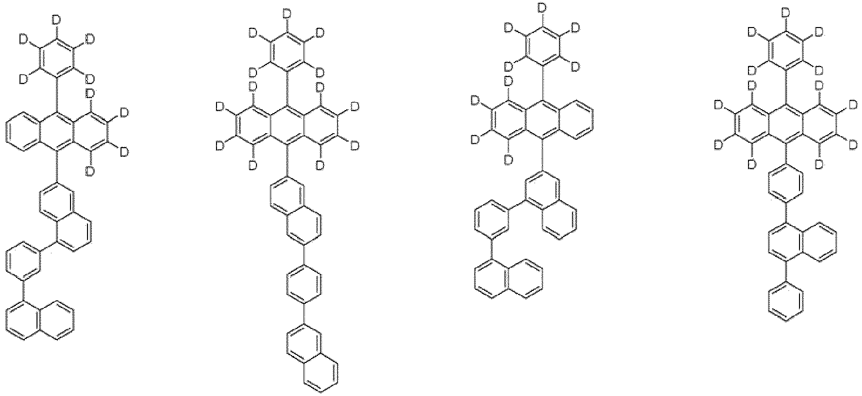

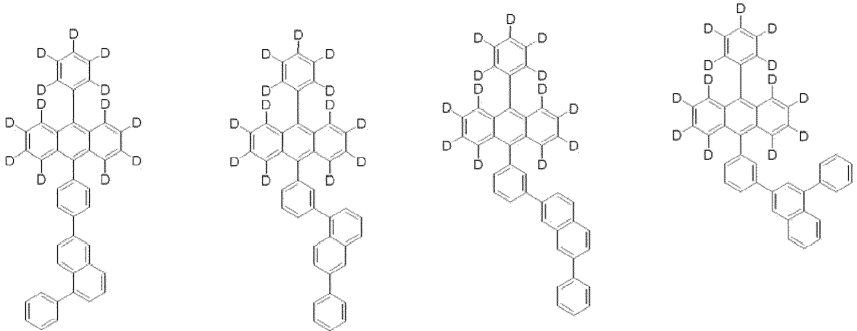

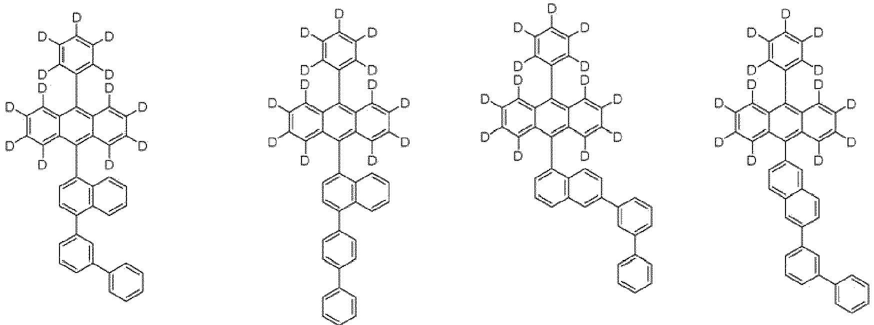

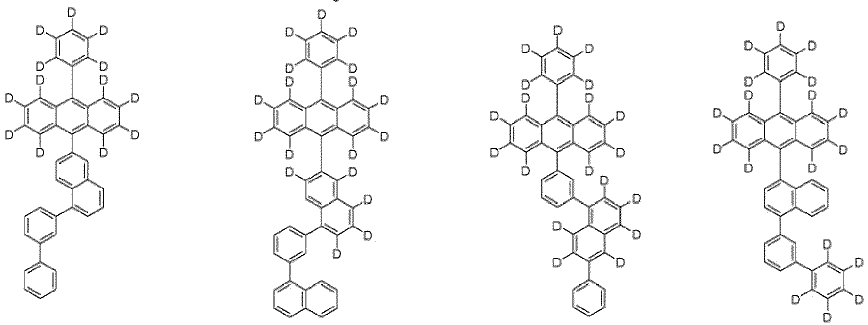

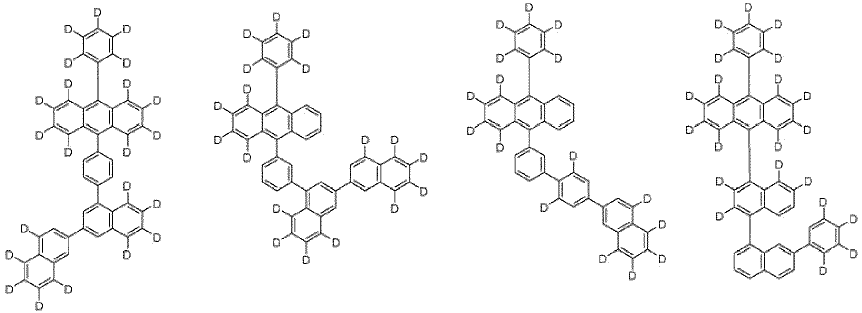

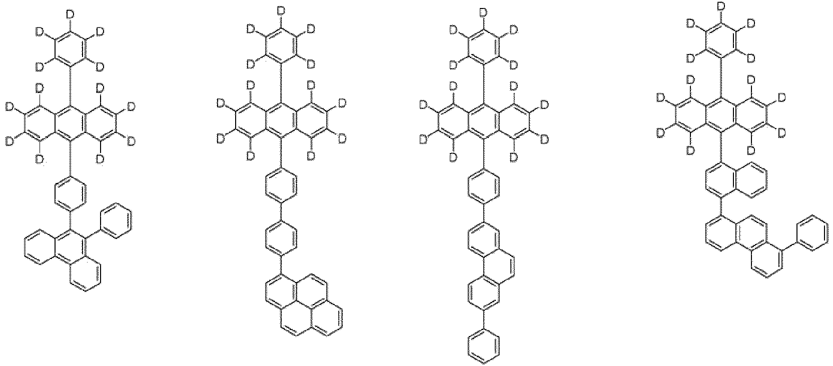

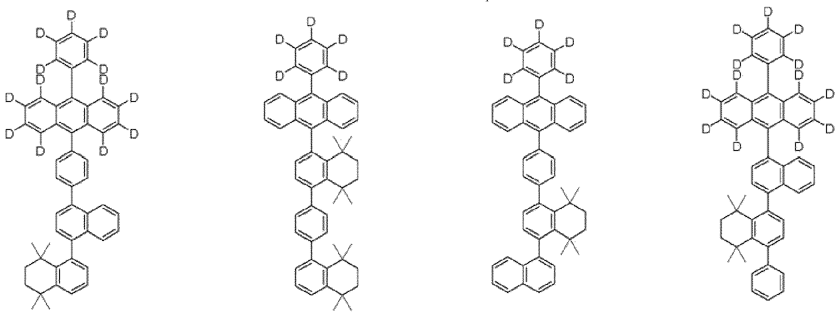

6. An organic light emitting device comprising a first electrode, a second electrode, and one or more organic layers interposed between the first and second electrodes wherein one of the organic layers is a light emitting layer, the light emitting layer comprises one or more hosts and a dopant, the hosts comprise at least one anthracene compound represented by Formula A: 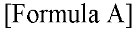 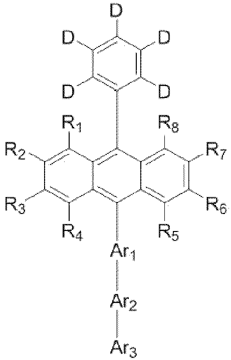
wherein R₁ to R₈ are identical to or different from each other and are each independently hydrogen or deuterium and Ar₁ to Ar₃ are identical to or different from each other and are each independently substituted or unsubstituted C₆-C₅₀ aryl or substituted or unsubstituted C₃-C₅₀ mixed aliphatic-aromatic cyclic group; and
at least one anthracene compound represented by Formula B: 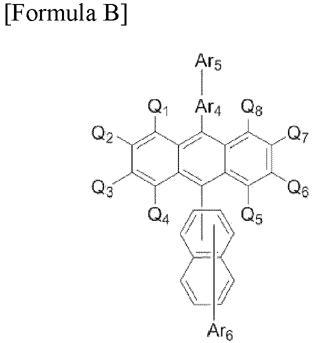
wherein Ar₄ to Ar₆ are identical to or different from each other and are each independently selected from hydrogen, deuterium, substituted or unsubstituted C₁-C₃₀ alkyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted amine, substituted or unsubstituted silyl, substituted or unsubstituted germanium, nitro, cyano, and halogen, and Q₁ to Q₈ are identical to or different from each other and are each independently hydrogen or deuterium (with the proviso that at least one of Q₁ to Q₈ is deuterium), the "substituted" in the definitions of Ar₁ to Ar₃ in Formula A and Ar₄ to Ar₆ in Formula B indicating substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof, the "unsubstituted" in the same definition indicating having no substituent, and one or more hydrogen atoms in each of the substituents being optionally replaced by deuterium atoms, and the anthracene compound represented by Formula A and the anthracene compound represented by Formula B are different from each other.

7. The organic light emitting device according to claim 1, wherein the compound represented by Formula B is selected from the following compounds:

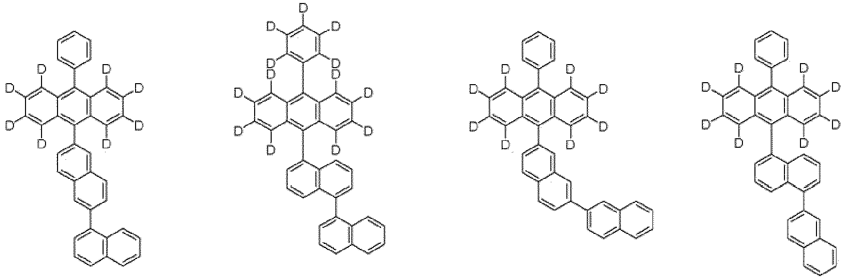

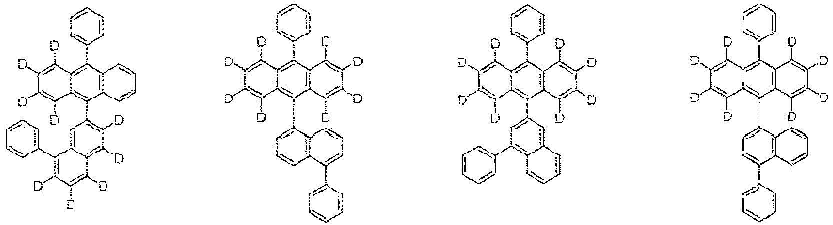

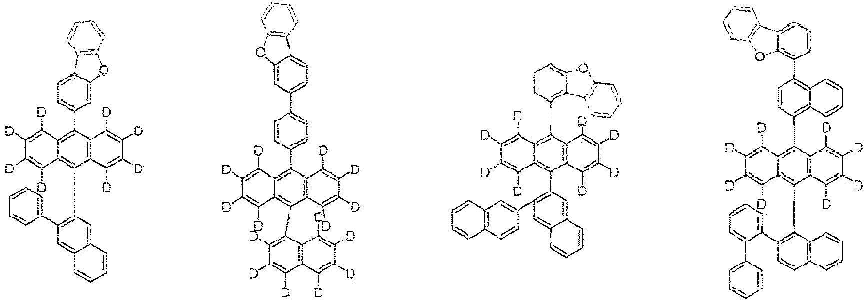

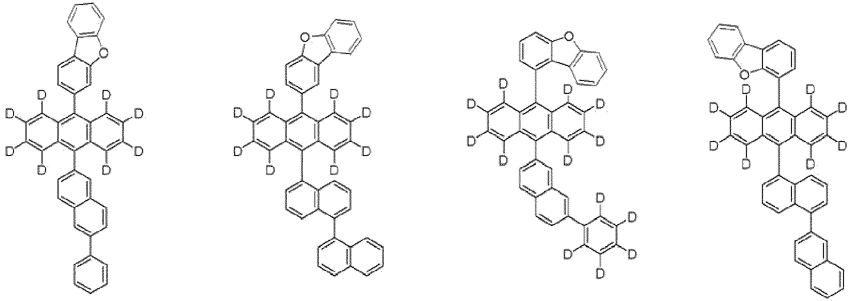

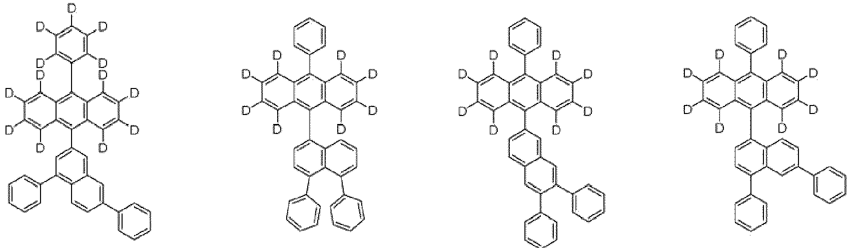

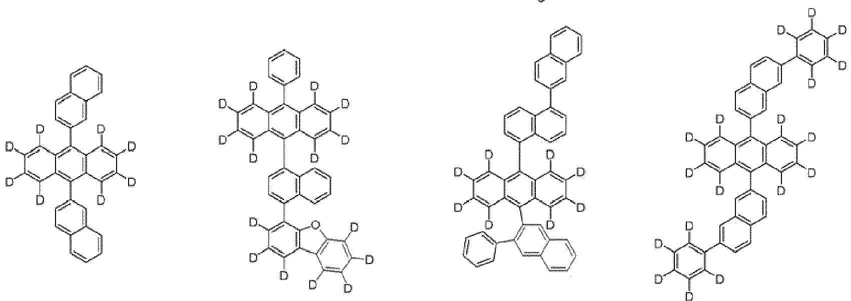

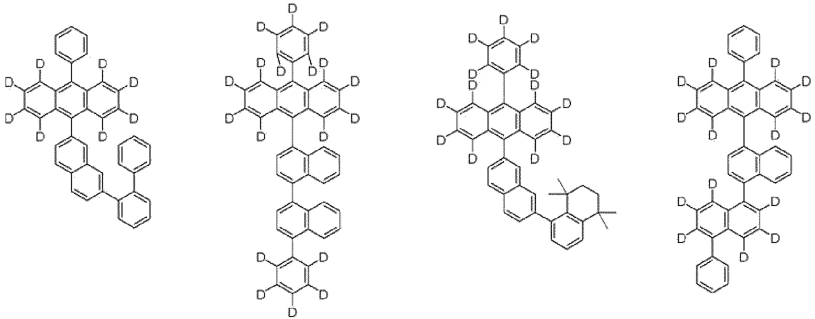

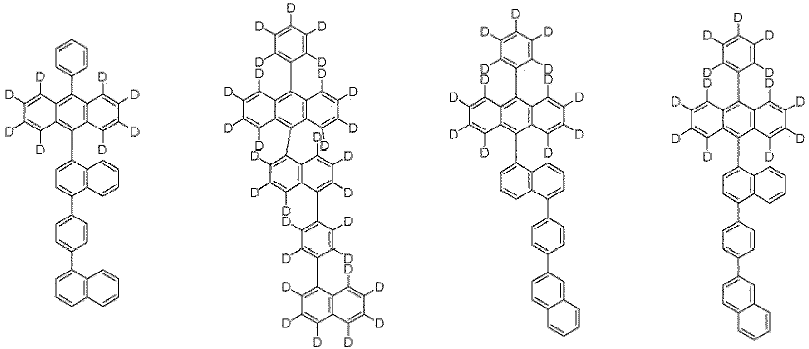

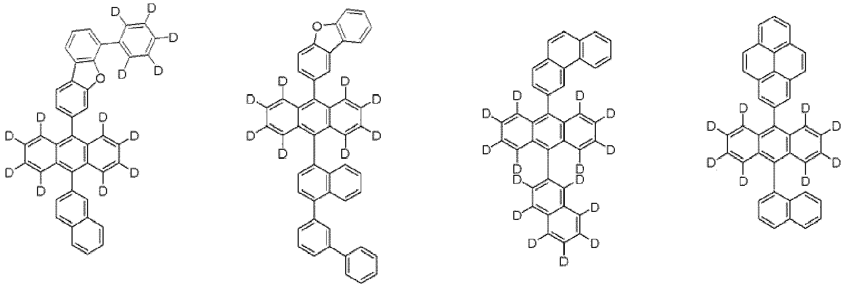

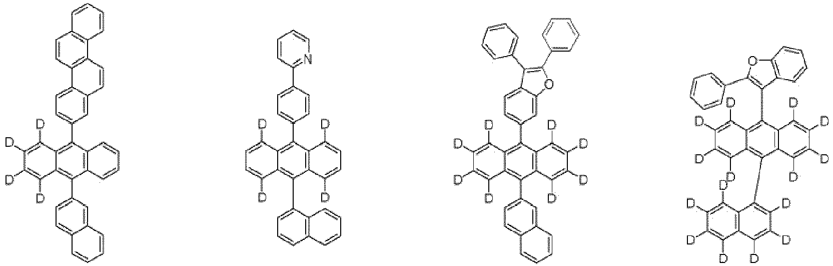

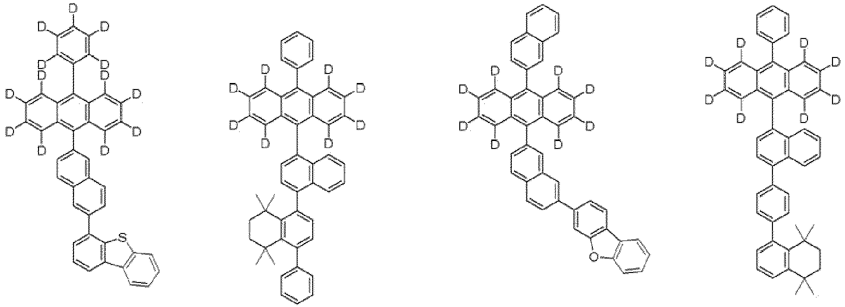

8. The organic light emitting device according to claim 6, wherein the dopant is a polycyclic compound represented by Formula 1: 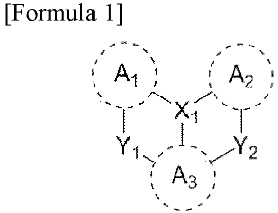 wherein X₁ is B, P=O, P=S or Al, Y₁ and Y₂ are identical to or different from each other and are each independently selected from NR₁₁, O, S, CR₁₂R₁₃, SiR₁₄R₁₅ or GeR₁₆R₁₇, A₁ to A₃ are identical to or different from each other and are each independently selected from substituted or unsubstituted C₆-C₅₀ aromatic hydrocarbon rings, substituted or unsubstituted C₃-C₅₀ aliphatic hydrocarbon rings, substituted or unsubstituted C₂-C₅₀ aromatic heterocyclic rings, substituted or unsubstituted C₂-C₅₀ aliphatic heterocyclic rings, and substituted or unsubstituted C₃-C₅₀ mixed aliphatic-aromatic cyclic group, R₁₁ to R₁₇ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, with the proviso that each of R₁₁ to R₁₇ is optionally linked to one or more of the rings A₁ to A₃ to form an alicyclic or aromatic monocyclic or polycyclic ring, R₁₂ and R₁₃ are optionally linked to each other to form an alicyclic or aromatic monocyclic or polycyclic ring, R₁₄ and R₁₅ are optionally linked to each other to form an alicyclic or aromatic monocyclic or polycyclic ring, and R₁₆ and R₁₇ are optionally linked to each other to form an alicyclic or aromatic monocyclic or polycyclic ring, the "substituted" in the definitions of Y₁, Y₂, and A₁ to A₃ in Formula 1 indicating substitution with one or more substituents selected from deuterium, C₁-C₂₄ alkyl, C₁-C₂₄ haloalkyl, C₃-C₂₄ cycloalkyl, C₂-C₂₄ alkenyl, C₂-C₂₄ alkynyl, C₁-C₂₄ heteroalkyl, C₂-C₂₄ heterocycloalkyl, C₆-C₃₀ aryl, C₇-C₃₀ arylalkyl, C₇-C₃₀ alkylaryl, C₂-C₃₀ heteroaryl, C₃-C₃₀ heteroarylalkyl, C₃-C₃₀ alkylheteroaryl, C₃-C₃₀ mixed aliphatic-aromatic cyclic groups, C₁-C₂₄ alkoxy, C₆-C₂₄ aryloxy, C₆-C₂₄ arylthionyl, C₁-C₄₀ amine, C₁-C₄₀ silyl, C₁-C₄₀ germanium, cyano, halogen, hydroxyl, and nitro, or a combination thereof, the "unsubstituted" in the same definition indicating having no substituent, and one or more hydrogen atoms in each of the substituents being optionally replaced by deuterium atoms.

9. The organic light emitting device according to claim 8, wherein the compound represented by Formula 1 is a polycyclic compound represented by Formula 2 or 3: 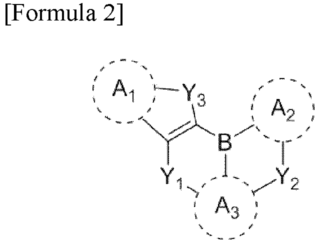
wherein Y₃ is O, S or NR₁₈, R₁₈ is as defined for R₁₁ to R₁₇ in Formula 1, and Y₁, Y₂, and A₁ to A₃ are as defined in Formula 1; 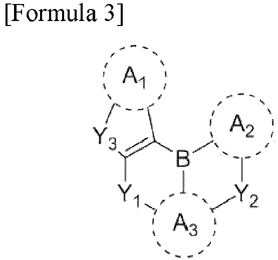
wherein Y₃ is O, S or NR₁₈, R₁₈ is as defined for R₁₁ to R₁₇ in Formula 1, and Y₁, Y₂, and A₁ to A₃ are as defined in Formula 1.

10. The organic light emitting device according to claim 8, wherein the compound represented by Formula 1 is selected from polycyclic compounds represented by Formulas 2-1, 2-2, 2-3, 3-1, 3-2, and 3-3: 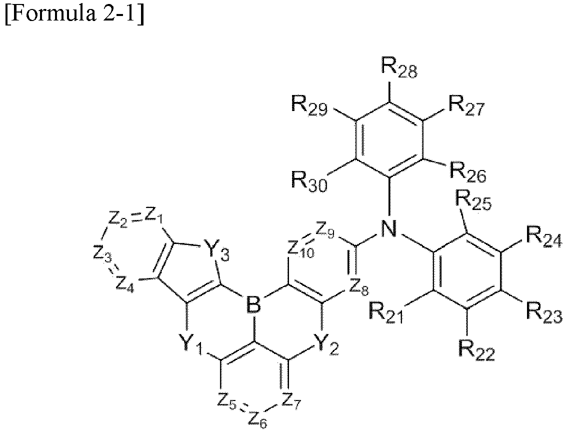
wherein Z₁ to Z₁₀ are identical to or different from each other and are each independently CR₃₁ or N, provided that when two or more of Z₁ to Z₁₀ are CR₃₁, the moieties CR₃₁ are identical to or different from each other, Y₃ is O, S or NR₁₈, R₁₈ is as defined for R₁₁ to R₁₇ in Formula 1, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, R₂₁ to R₃₀ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₂₁ to R₃₁ are optionally replaced by deuterium atoms, adjacent ones of the substituents of R₂₁ to R₃₀ are optionally linked to each other to form an alicyclic or aromatic monocyclic or polycyclic ring, and the groups R₃₁ are optionally linked to each other to form an alicyclic or aromatic monocyclic or polycyclic ring, provided that when Z₈ and Z₉ are CR₃₁, each of R₂₁, R₂₅, R₂₆, and R₃₀ adjacent to Z₈ and Z₉ is optionally bonded to R₃₁ to form an alicyclic or aromatic monocyclic or polycyclic ring, Y₁ and Y₂ are identical to or different from each other and are each independently as defined in Formula 1 or are each independently a linker represented by Structural Formula A: 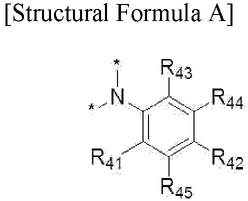
wherein R₄₁ to R₄₅ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₄₁ to R₄₅ are optionally replaced by deuterium atoms and each of R₄₁ to R₄₅ is optionally linked to one or more adjacent substituents to form an alicyclic or aromatic monocyclic or polycyclic ring; 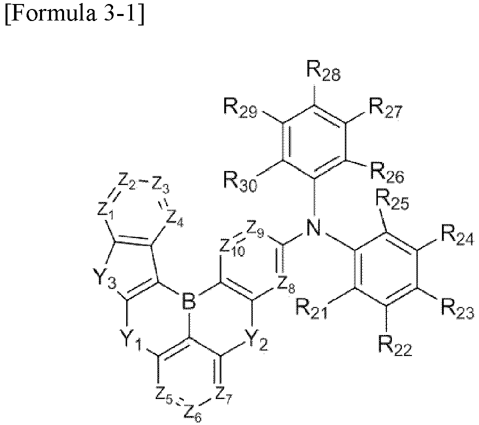
wherein Z₁ to Z₁₀, Y₁ to Y₃, and R₂₁ to R₃₀ are as defined in Formula 2-1; 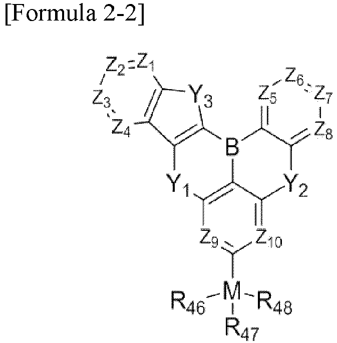
wherein Z₁ to Z₁₀ are identical to or different from each other and are each independently CR₃₁ or N, provided that when two or more of Z₁ to Z₁₀ are CR₃₁, adjacent ones of the moieties CR₃₁ are identical to or different from each other and when two or more of Z₁ to Z₈ are CR₃₁, adjacent ones of the groups R₃₁ are optionally linked to each other to form an alicyclic or aromatic monocyclic or polycyclic ring, Y₃ is O, S or NR₁₈, R₁₈ is as defined for R₁₁ to R₁₇ in Formula 1, M is Si or Ge, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, R₄₆ to R₄₈ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₃₁ and R₄₆ to R₄₈ are optionally replaced by deuterium atoms and the substituents of R₄₆ to R₄₈ are optionally linked to each other to form an alicyclic or aromatic monocyclic or polycyclic ring; 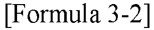 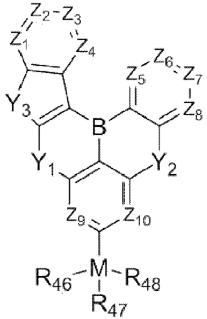
wherein Z₁ to Z₁₀, Y₁ to Y₃, M, and R₄₆ to R₄₈ are as defined in Formula 2-2; 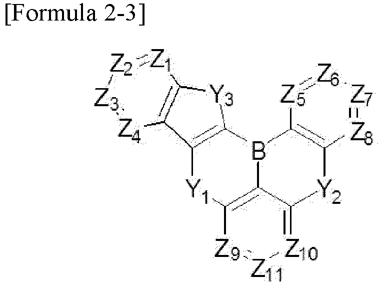
wherein Z₁ to Z₁₁ are identical to or different from each other and are each independently CR₃₁ or N, provided that when two or more of Z₁ to Z₁₁ are CR₃₁, adjacent ones of the moieties CR₃₁ are identical to or different from each other, Y₃ is O, S or NR₁₈, R₁₈ is as defined for R₁₁ to R₁₇ in Formula 1, the groups R₃₁ are identical to or different from each other and are each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₅₀ cycloalkyl, substituted or unsubstituted C₂-C₅₀ heterocycloalkyl, substituted or unsubstituted C₂-C₅₀ heteroaryl, substituted or unsubstituted C₃-C₅₀ mixed aliphatic-aromatic cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, substituted or unsubstituted germanium, nitro, cyano, and halogen, with the proviso that one or more hydrogen atoms in each of the substituents of the groups R₃₁ are optionally replaced by deuterium atoms, Y₁ and Y₂ are identical to or different from each other and are each independently as defined in Formula 1 or are each independently a linker represented by Structural Formula B: 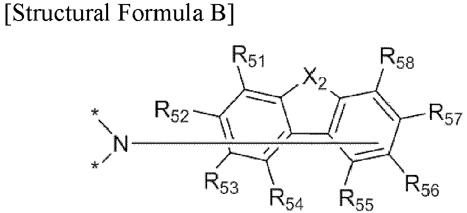
wherein X₂ is O or S, R₅₁ to R₅₈ are identical to or different from each other and are each independently selected from hydrogen, deuterium, substituted or unsubstituted C₁-C₂₄ alkyl, substituted or unsubstituted C₂-C₂₄ alkynyl, substituted or unsubstituted C₂-C₂₄ alkenyl, substituted or unsubstituted C₆-C₃₀ aryl, substituted or unsubstituted C₃-C₂₄ cycloalkyl, substituted or unsubstituted C₂-C₂₄ heterocycloalkyl, substituted or unsubstituted C₂-C₃₀ heteroaryl, substituted or unsubstituted C₃-C₃₀ mixed aliphatic-aromatic cyclic groups, substituted or unsubstituted C₁-C₂₄ alkoxy, substituted or unsubstituted C₆-C₂₄ aryloxy, substituted or unsubstituted C₁-C₂₄ alkylthioxy, substituted or unsubstituted C₅-C₂₄ arylthioxy, substituted or unsubstituted C₁-C₃₀ amine, substituted or unsubstituted C₁-C₃₀ silyl, substituted or unsubstituted C₁-C₃₀ germanium, nitro, cyano, and halogen, with the proviso that one or more hydrogen atoms in each of the substituents of R₅₁ to R₅₈ are optionally replaced by deuterium atoms, one of R₅₁ to R₅₈ forms a single bond with the nitrogen atom, and each of the others is optionally linked to one or more adjacent substituents to form an alicyclic or aromatic monocyclic or polycyclic ring; and 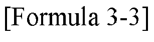 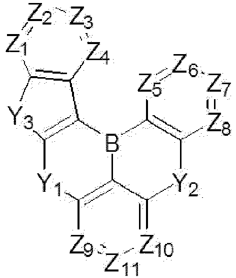
wherein Z₁ to Z₁₁ and Y₁ to Y₃ are as defined in Formula 2-3, the "substituted" in the definitions of Z₁ to Z₁₀, Y₁ to Y₃, and R₂₁ to R₃₀ in Formula 2-1, Z₁ to Z₁₀, Y₁ to Y₃, and R₄₆ to R₄₈ in Formula 2-2, Z₁ to Z₁₁ and Y₁ to Y₃ in Formula 2-3, Z₁ to Z₁₀, Y₁ to Y₃, and R₂₁ to R₃₀ in Formula 3-1, Z₁ to Z₁₀, Y₁ to Y₃, and R₄₆ to R₄₈ in Formula 3-2, and Z₁ to Z₁₁ and Y₁ to Y₃ in Formula 3-3 indicating substitution with one or more substituents selected from deuterium, C₁-C₁₈ alkyl, C₁-C₁₈ haloalkyl, C₃-C₁₈ cycloalkyl, C₂-C₁₈ alkenyl, C₂-C₁₈ alkynyl, C₁-C₁₈ heteroalkyl, C₂-C₁₈ heterocycloalkyl, C₆-C₂₄ aryl, C₇-C₂₄ arylalkyl, C₇-C₂₄ alkylaryl, C₂-C₂₄ heteroaryl, C₃-C₂₄ heteroarylalkyl, C₃-C₂₄ alkylheteroaryl, C₃-C₂₄ mixed aliphatic-aromatic cyclic groups, C₁-C₁₈ alkoxy, C₆-C₁₈ aryloxy, C₆-C₁₈ arylthionyl, C₁-C₃₀ amine, C₁-C₃₀ silyl, C₁-C₃₀ germanium, cyano, halogen, hydroxyl, and nitro, or a combination thereof, the "unsubstituted" in the same definition indicating having no substituent, and one or more hydrogen atoms in each of the substituents being optionally replaced by deuterium atoms.

11. The organic light emitting device according to claim 8, wherein the polycyclic compound represented by Formula 1 is selected from the following compounds:

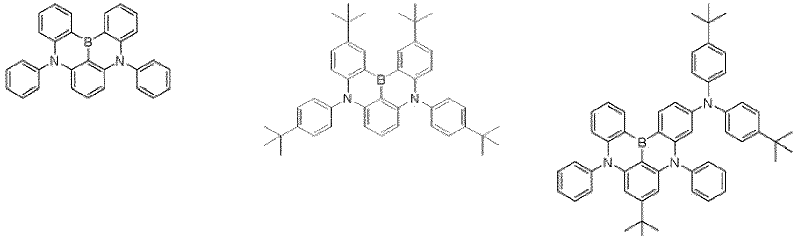

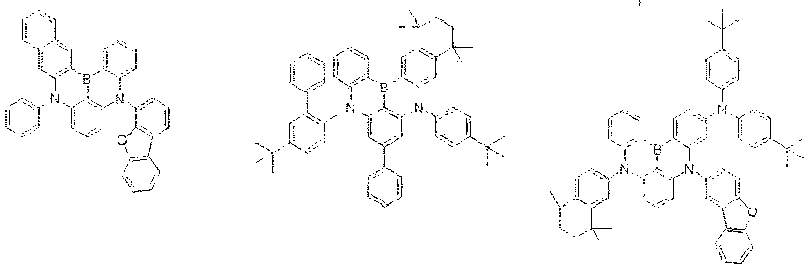

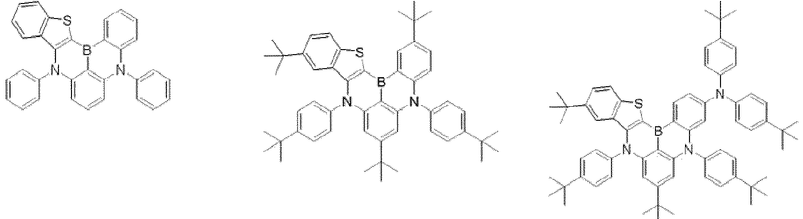

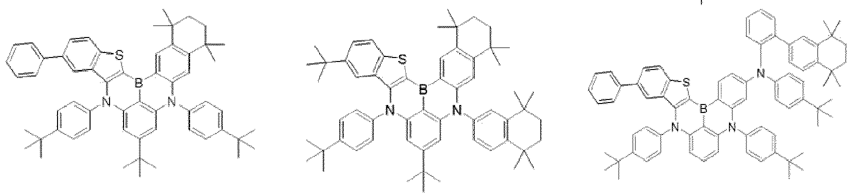

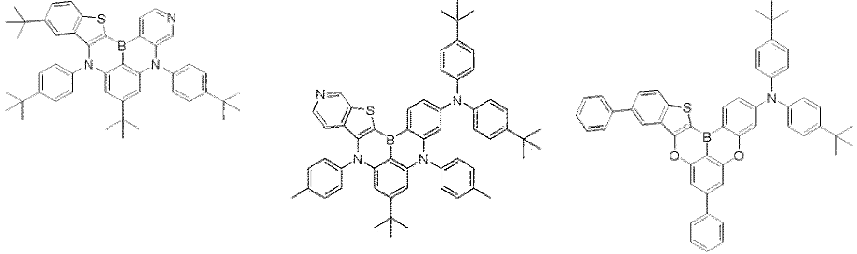

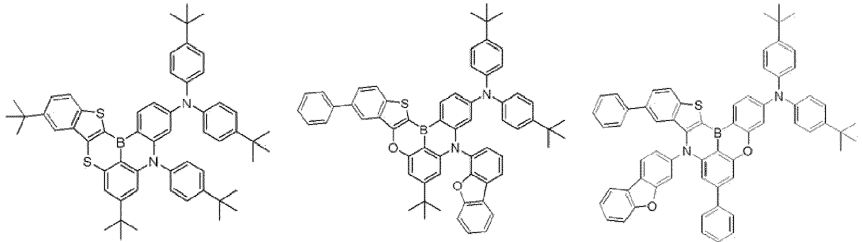

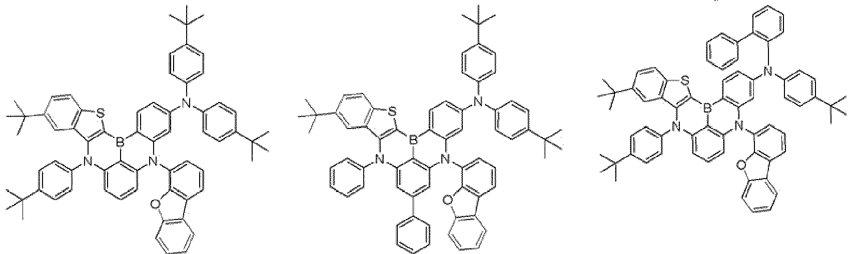

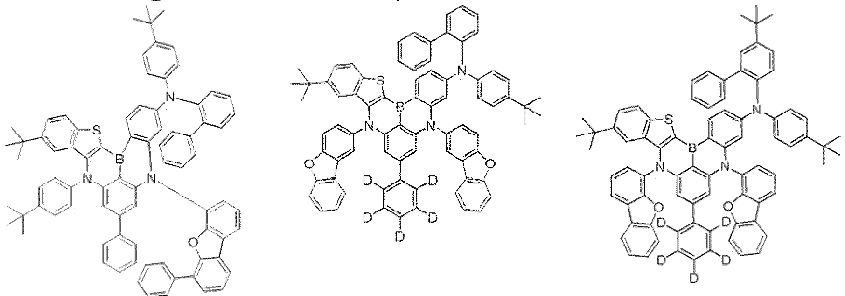

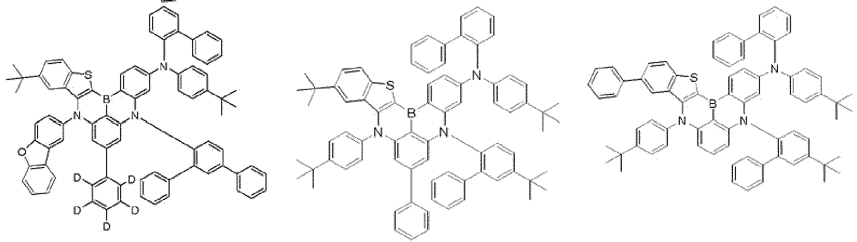

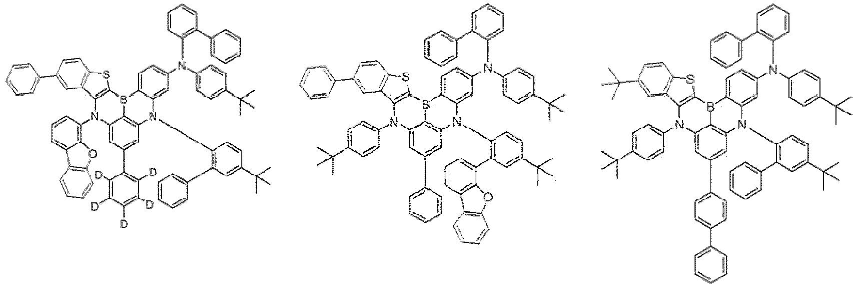

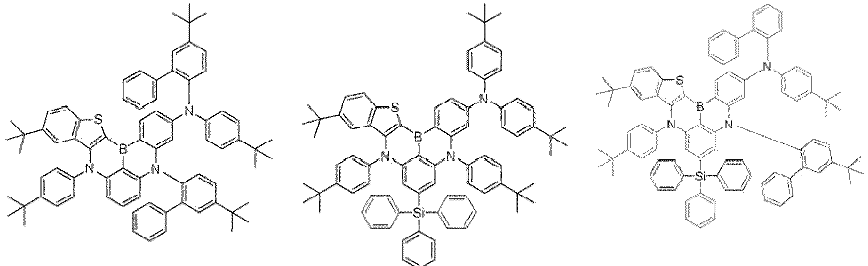

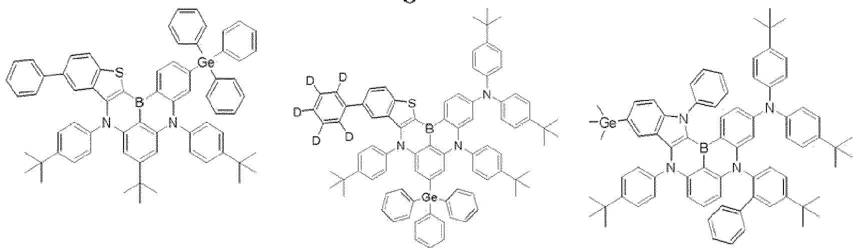

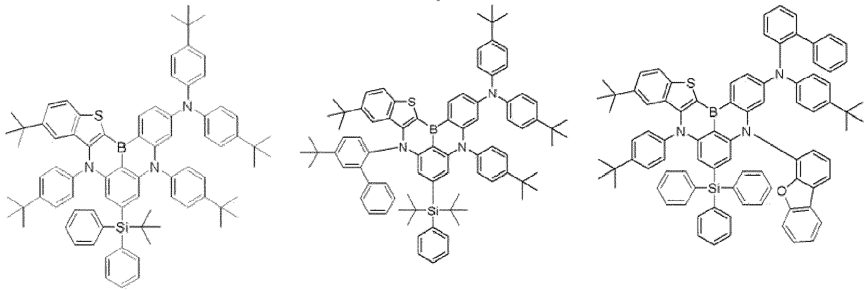

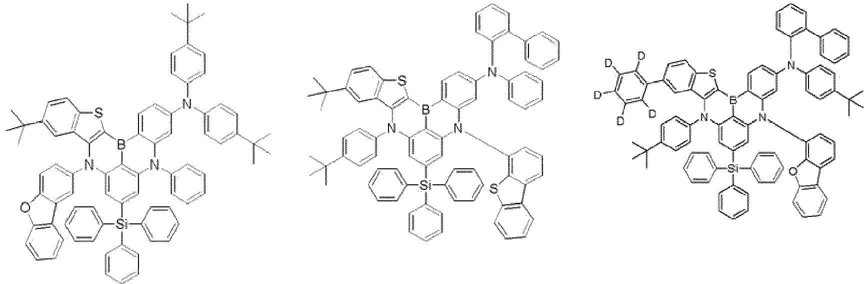

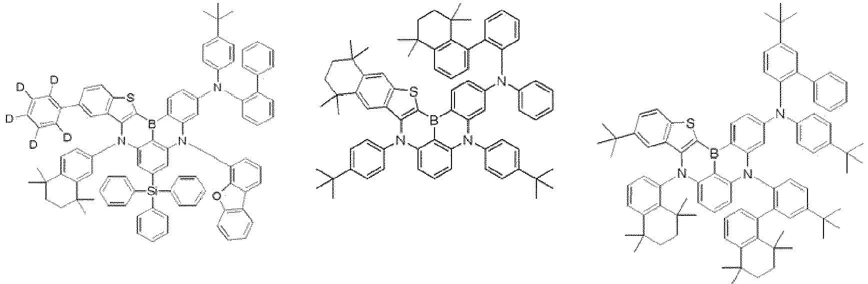

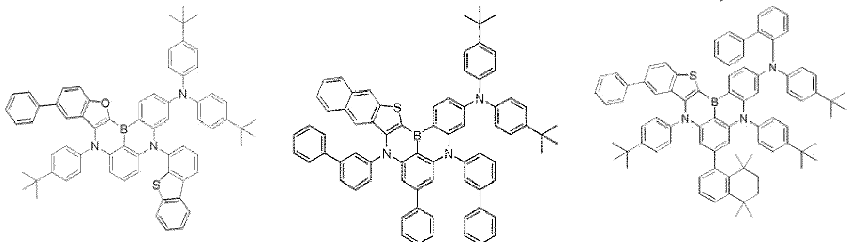

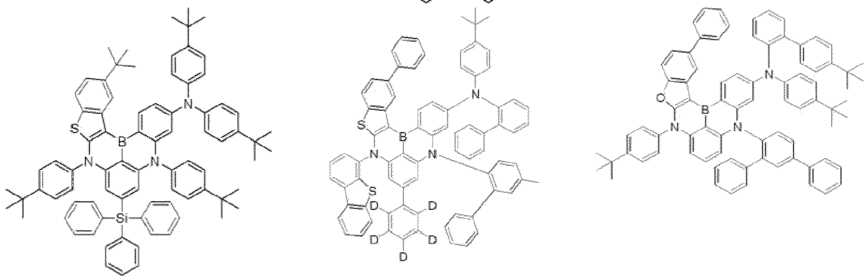

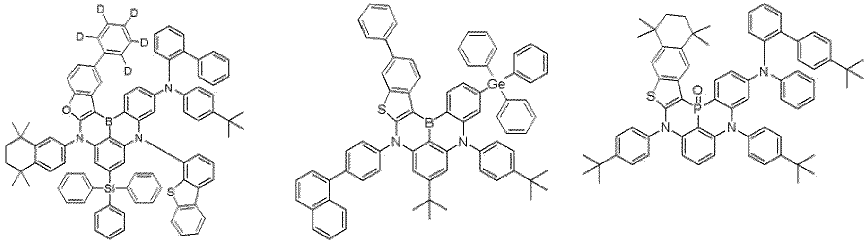

12. The organic light emitting device according to claim 6, wherein the organic layers comprise a hole injecting layer, a hole transport layer, an electron blocking layer, a functional layer having functions of both hole injection and hole transport, a light emitting layer, an electron transport layer, an electron injecting layer, a hole blocking layer, and/or a functional layer having functions of both electron injection and electron transport.

13. The organic light emitting device according to claim 12, wherein each of the organic layers is formed by a deposition or solution process.

14. The organic light emitting device according to claim 8, wherein one or more dopants other than the compound represented by Formula 1 are mixed or stacked in the light emitting layer.

15. The organic light emitting device according to claim 6, wherein the organic light emitting device is used in a display or lighting system selected from flat panel displays, flexible displays, monochromatic flat panel lighting systems, white flat panel lighting systems, flexible monochromatic lighting systems, flexible white lighting systems, displays for automotive applications, displays for virtual reality, and displays for augmented reality.
